# EUROPEAN PATENT APPLICATION

(11) **EP 4 464 710 A1**
(43) Date of publication of application: **20.11.2024**
(21) Application number: 23740053.6
(22) Date of filing: 11.01.2023
(51) Int. Cl.: C07D 513/16, C07D 519/00, C07D 471/16, A61K 31/55, A61K 31/4353, A61P 25/24

(54) **CYCLOPIPERIDINE COMPOUND, PREPARATION METHOD THEREFOR, AND USE THEREOF**

(30) Priority: 14.01.2022 CN 202210041795
(71) Applicant: ShanghaiTech University, Shanghai 201210 (CN); Center for Excellence in Molecular Cell Science, Chinese Academy of Sciences, Shanghai 200031 (CN)
(72) Inventor: CHENG, Jianjun, Shanghai 201210 (CN); WANG, Sheng, Shanghai 200031 (CN); WANG, Huan, Shanghai 201210 (CN); CAO, Dongmei, Shanghai 200031 (CN); YU, Jing, Shanghai 200031 (CN); DUAN, Wenwen, Shanghai 201210 (CN); HE, Licong, Shanghai 200031 (CN)
(74) Representative: Dehns
(86) International application number: PCT/CN2023/071816
(87) International publication number: WO 2023/134711

(57) **Abstract**

Disclosed are a cyclopiperidine compound, a preparation method therefor, and a use thereof. The cyclopiperidine compound of the present invention is represented by formula I'. The cyclopiperidine compound of the present invention has a better effect for suppressing depression and has good application prospects.

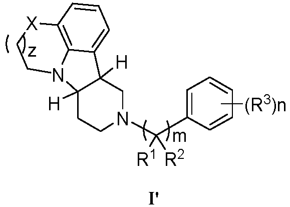

## Description

The present application claims the right of the priority of Chinese patent application 2022100417955 filed on January 14, 2022. The contents of the above Chinese patent application are incorporated herein by reference in their entireties.

### TECHNICAL FIELD

The present disclosure relates to a fused-piperidine compound, a preparation method therefor, and a use thereof.

### BACKGROUND

Depression is a serious mental disorder that severely impacts human health and affects approximately 300 million people globally, with tens of thousands of depressive patients committing suicide each year. Depression is characterized by a marked and persistent low mood, accompanied by symptoms, such as loss of interest, sleep disturbances, and low self-esteem, with severe cases presenting suicidal ideation or acts of self-harm and suicide.

Current known antidepressants primarily include selective serotonin reuptake inhibitors (SSRIs), tricyclic antidepressants, and monoamine oxidase inhibitors. SSRIs are widely used in clinical settings, mainly comprising sertraline, escitalopram, fluoxetine, paroxetine, and others. SSRIs function by inhibiting the reuptake of the central neurotransmitter serotonin (5-HT), thereby increasing the concentration of 5-HT in the synaptic cleft. Although SSRIs have the advantages of better tolerance and higher safety compared to traditional tricyclic antidepressants, they still have several obvious disadvantages: (1) slow onset of action, taking several weeks to months to become effective; (2) low response rate, with more than one-third of patients unresponsive; (3) adverse reactions such as nausea, weight gain, and sexual dysfunction are still quite common. Therefore, there is an urgent need to develop novel antidepressants.

At the beginning of this century, clinical trials unexpectedly discovered the rapid antidepressant effects of ketamine, a medical anesthetic. A single sub-anesthetic dose of ketamine can rapidly alleviate symptoms of depression within a few hours. In March 2019, the U.S. Food and Drug Administration (FDA) approved (*S*)-ketamine (esketamine, marketed as a nasal spray) for the treatment of patients with treatment-resistant depression (Swainson et al.; Expert Rev Neurother, 2019, 19(10): 899-911). In terms of mechanism of action, esketamine is a non-competitive and subtype non-selective activity-dependent *N*-methyl-D-aspartate (NMDA) receptor antagonist that functions by antagonizing NMDA function (Zhang et al.; Nature, 2021, 596(7871): 301-305). In depression models in mice, ketamine was able to remodel many synapses that had previously existed but had disappeared under prolonged stress. However, as an antidepressant, ketamine can cause side effects such as dissociative hallucinations, and carries addiction risks, and clinical application is limited.

Recent studies have also indicated that psychedelics hold great potential in treating diseases such as depression, anxiety, drug addiction, and post-traumatic stress disorder (PTSD) (Nutt et al., Cell, 2020, 181(1): 24-28). Classic psychedelics mainly include substances like psilocybin, lysergic acid diethylamide (LSD), *N*,*N*-dimethyltryptamine (DMT), and ibogaine. Among them, psilocybin has shown rapid onset of action and sustained relief of depression in phase II clinical trials, and multiple clinical trials using psilocybin for the rapid treatment of depression are currently underway. In terms of mechanism of action, psychedelics function by activating the 5-HT_{2A} receptor, but the mechanisms for discovering antidepressants without hallucinogenic effects by modulating the efficacy and signaling pathway selectivity of agonists remain unclear.

Lumateperone is a compound with a tetracyclic fused-piperidine parent skeleton 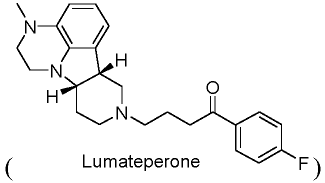 and was approved by the U.S. FDA in December 2019 for the treatment of schizophrenia in adults (Blair, Drugs, 80(4): 417-423). In December 2021, the U.S. FDA approved a new indication for lumateperone: as a monotherapy and as an adjunctive therapy with lithium or valproate salts for the treatment of depressive episodes associated with bipolar I or II disorder (bipolar depression) in adult patients (Calabrese; Am J Psychiatry, 2021, 178(12): 1098-1106).

In terms of mechanism of action, lumateperone functions as a potent antagonist of the 5-HT_{2A} receptor (Kᵢ = 0.54 nM), a postsynaptic D₂ receptor antagonist (Kᵢ = 32 nM), and an inhibitor of the serotonin transporter (SERT) (Kᵢ = 61 nM) (Li et al.; J Med Chem 2014, 57, 2670-2682). As an antidepressant, lumateperone is only used for depressive episodes associated with bipolar I or II disorder, with a patient response rate of 51.1% (Calabrese et al., Am J Psychiatry 2021, 178:12); its effects on patients suffering from general depression and treatment-resistant depression have not yet been reported. Its antidepressant effects and mechanism of action are similar to those of antipsychotic drugs such as lurasidone, quetiapine, and cariprazine (Calabrese et al., Am J Psychiatry 2021, 178:12).

### SUMMARY OF THE INVENTION

The present invention aims to overcome the drawbacks of limited variety or hallucinogenic side effects of antidepressants in the prior art, and provides a fused-piperidine compound, a preparation method therefor, and a use thereof. The fused-piperidine compound of the present disclosure exhibits good antidepressant effects and further avoids hallucinogenic side effects.

The present disclosure overcomes the above technical problems through the following technical solutions.

The present disclosure provides a fused-piperidine compound of formula I' or a pharmaceutically acceptable salt thereof: 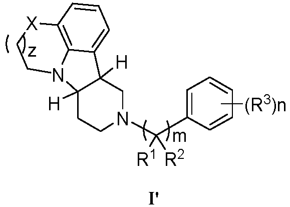
wherein X is 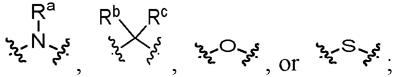
R^{a}, R^{b}, and R^{c} are independently hydrogen or C₁-C₄ alkyl;
R¹ and R² are independently hydrogen, halogen, or C₁-C₄ alkyl;
R³ is independently hydrogen, halogen, hydroxyl, or C₁-C₄ alkoxy;
m is 1, 2, or 3;
n is 1, 2, or 3;
z is 1 or 2.

In one embodiment, the fused-piperidine compound of formula I' is not

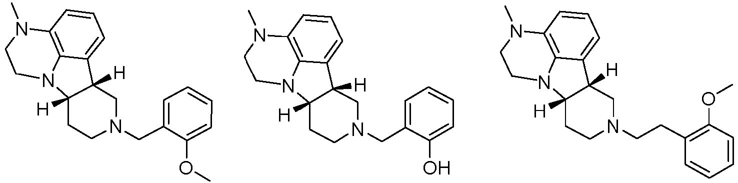

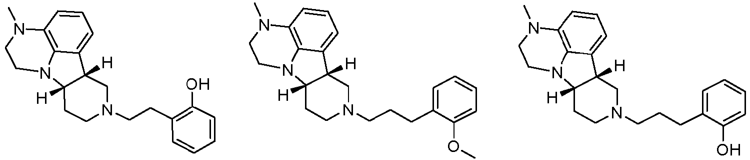

In one embodiment, the fused-piperidine compound of formula I' is a fused-piperidine compound of formula I-a, 1-b, I, or I-c: 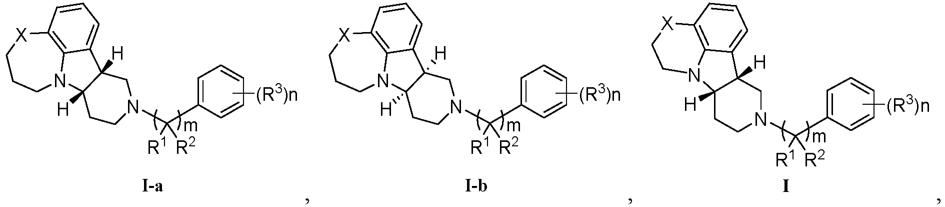 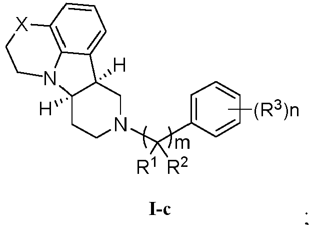 wherein X, R¹, R², R³, m, and n are as defined above.

In one embodiment, the fused-piperidine compound of formula I' is a fused-piperidine compound of formula I: 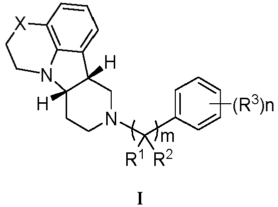
wherein X is 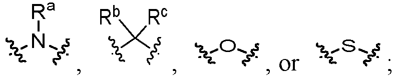
R^{a}, R^{b}, and R^{c} are independently hydrogen or C₁-C₄ alkyl;
R¹ and R² are independently hydrogen, halogen, or C₁-C₄ alkyl;
R³ is independently hydrogen, halogen, hydroxyl, or C₁-C₄ alkoxy;
m is 1, 2, or 3;
n is 1, 2, or 3.

In one embodiment, in R^{a}, R^{b}, and R^{c}, the C₁-C₄ alkyl is independently and preferably methyl, ethyl, *n*-propyl, isopropyl, *n*-butyl, isobutyl, *sec*-butyl, or *tert*-butyl, for example, methyl.

In one embodiment, in R¹ and R², the halogen is independently and preferably F, Cl, Br, or I.

In one embodiment, in R¹ and R², the C₁-C₄ alkyl is independently and preferably methyl, ethyl, *n*-propyl, isopropyl, *n*-butyl, isobutyl, *sec*-butyl, or *tert*-butyl.

In one embodiment, in R³, the halogen is preferably F, Cl, Br, or I.

In one embodiment, in R³, the C₁-C₄ alkoxy is preferably methoxy, ethoxy, n-propoxy, isopropoxy, *n*-butoxy, isobutoxy, *sec*-butoxy, or *tert*-butoxy, for example, methoxy.

In one embodiment, the substitution position of R³ on the benzene ring is *ortho-*position, *"ortho-* and *para*-position", or *"ortho-* and *meta*-position", wherein *ortho*-position, *para*-position, or *meta*-position refers to the substitution position of R³ on the benzene ring relative to 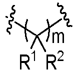 in formula **I'.**

In one embodiment, the substitution position of R³ on the benzene ring is *ortho-*position, *"ortho-* and *para*-position", or *"ortho-* and meta-position", wherein *ortho*-position, *para*-position, or *meta*-position refers to the substitution position of R³ on the benzene ring relative to 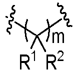 in formula I.

In one embodiment, X is

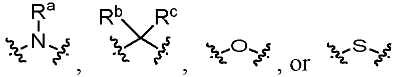

In one embodiment, X is 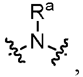 and R^{a} is C₁-C₄ alkyl.

In one embodiment, X is

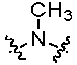

In one embodiment, X is 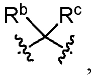 and R^{b} and R^{c} are independently hydrogen.

In one embodiment, X is

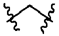

In one embodiment, R¹ and R² are hydrogen.

In one embodiment, R³ is independently hydrogen, hydroxyl, or C₁-C₄ alkoxy.

In one embodiment, n is 1 or 2.

In one embodiment, X is

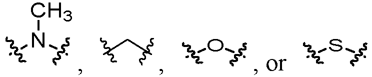

In one embodiment, 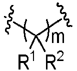 is

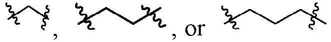

In one embodiment, 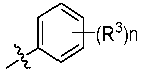 is

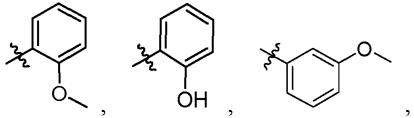

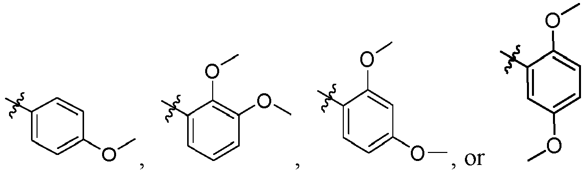

In one embodiment, 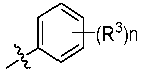 is

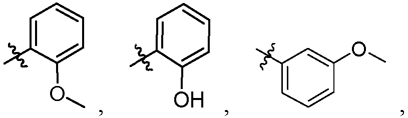

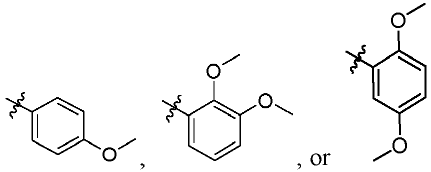

In one embodiment, 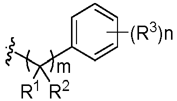 is

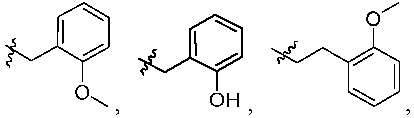

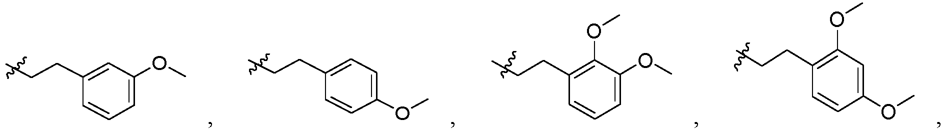

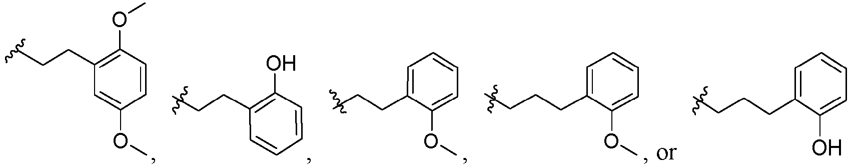

In one embodiment, 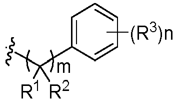 is

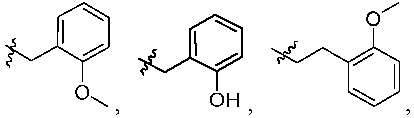

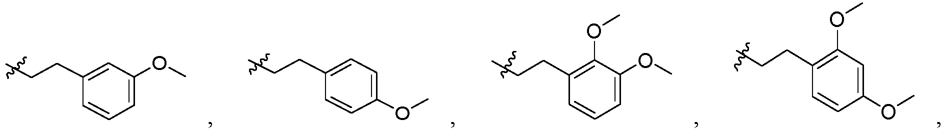

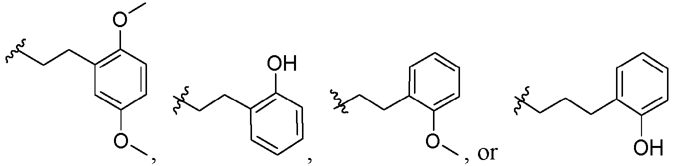

In one embodiment, X is 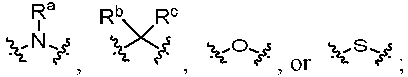
R^{a} is C₁-C₄ alkyl;
R^{b} and R^{c} are independently hydrogen;
R¹ and R² are hydrogen;
R³ is independently hydrogen, hydroxyl, or C₁-C₄ alkoxy;
n is 1 or 2;
z is 1 or 2;
the substitution position of R³ on the benzene ring is *ortho*-position, *"ortho-* and *para-*position", or *"ortho-* and *meta*-position".

In one embodiment, X is 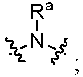
R^{a} is C₁-C₄ alkyl;
R¹ and R² are hydrogen;
R³ is independently hydrogen, hydroxyl, or C₁-C₄ alkoxy;
n is 1 or 2;
the substitution position of R³ on the benzene ring is *ortho*-position, *"ortho-* and *para-*position", or *"ortho-* and *meta*-position".

In one embodiment, the fused-piperidine compound of formula **I'** is any one of the following compounds:

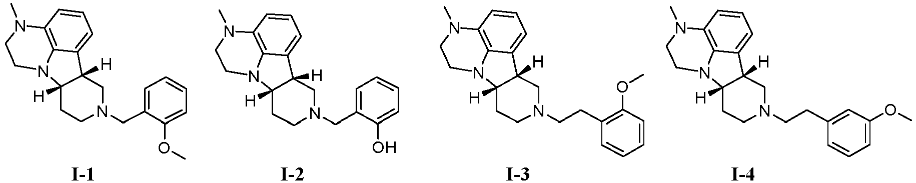

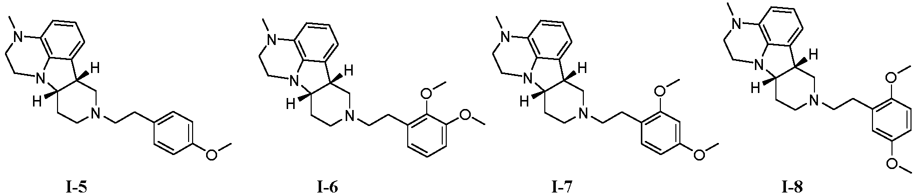

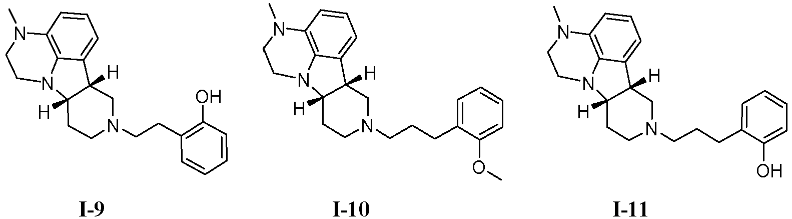

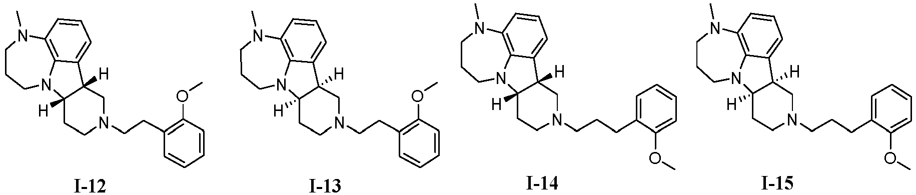

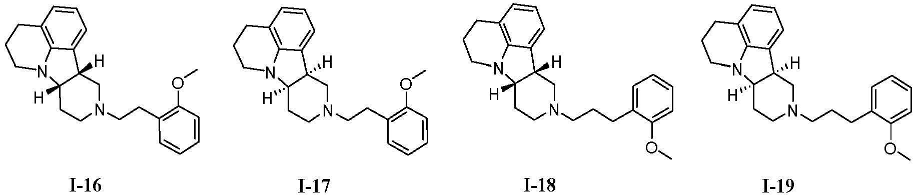

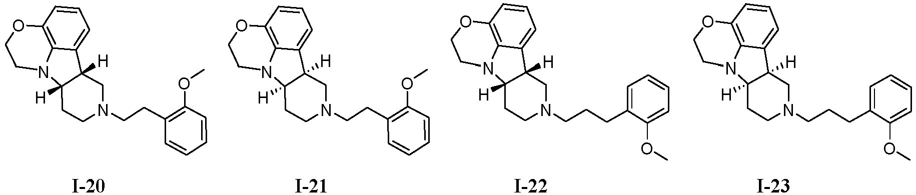

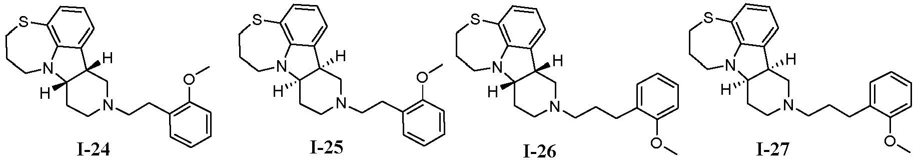

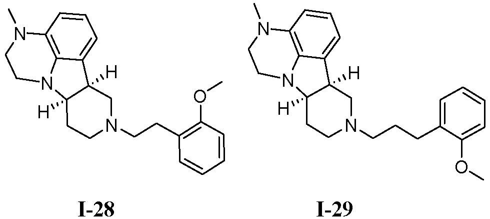

The present disclosure further provides a method for preparing the fused-piperidine compound of formula **I'** or a pharmaceutically acceptable salt thereof, which includes Method **1,** Method **2,** or Method **3:**
when R³ is independently hydrogen, halogen, or C₁-C₄ alkoxy, the method for preparing the fused-piperidine compound of formula **I'** or the salt thereof is Method **1** or Method **2;**
Method **1** comprises the following steps: in the presence of a base, conducting a substitution reaction in a solvent between a compound of formula **II**' and a compound of formula **III** to obtain the fused-piperidine compound of formula **I';** 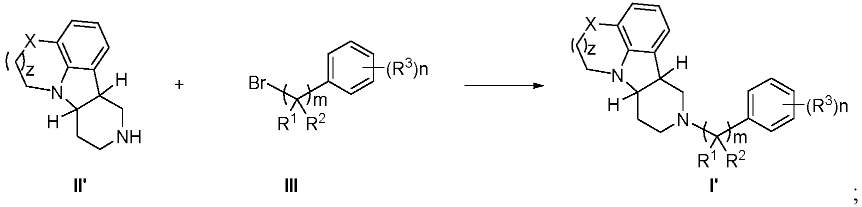
Method **2** comprises the following steps: in the presence of a reducing agent, conducting a reductive amination reaction in a solvent between a compound of formula **II**' and a compound of formula **IV** to obtain the fused-piperidine compound of formula **I';** 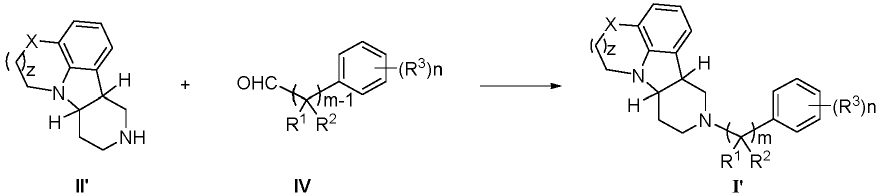
when R³ is independently hydroxyl, the method for preparing the fused-piperidine compound of formula **I'** or the salt thereof is Method **3;**
Method **3** comprises the following steps: in the presence of BBr₃, reacting the fused-piperidine compound of formula **I'** as described in Method **1** or **2** in a solvent to obtain the fused-piperidine compound of formula **I'** *(i.e.,* R³ is hydroxyl);
in Methods **1, 2,** and **3,** X, R^{a}, R^{b}, R^{c}, R¹, R², m, and n are the same as defined above;
"m-1" refers to a value that is one carbon atom less than "m".

In Method **1,** the conditions and procedures for the substitution reaction may be those conventional for such reactions in the art. The following conditions and procedures are particularly preferred in the present disclosure:
The base is preferably an organic base, for example, diisopropylethylamine (DIPEA). The molar ratio of the base to the compound of formula **II**' is preferably (1 to 3):1, for example, 1:1.

The molar ratio of the compound of formula **III** to the compound of formula **II**' is preferably (1 to 3): 1, for example, 1.5:1.

The solvent is preferably a polar aprotic organic solvent, for example, dimethyl sulfoxide (DMSO).

The temperature for the substitution reaction is preferably room temperature to 80°C, more preferably 50°C to 80°C, for example, 60°C.

In Method 2, the conditions and procedures for the reaction may be those conventional for such reactions in the art. The following conditions and procedures are particularly preferred in the present disclosure:

The solvent is preferably an alcoholic solvent, for example, methanol.

The reducing agent is preferably a borane-based reducing agent, for example, sodium cyanoborohydride.

The temperature for the reductive amination reaction is preferably 0 to 60°C, for example, room temperature.

In Method **3,** the conditions and procedures for the reaction may be those conventional for such reactions in the art. The following conditions and procedures are particularly preferred in the present disclosure:

The solvent is preferably a halogenated hydrocarbon organic solvent, for example, dichloromethane.

The temperature for the reaction is preferably -40°C to 40°C; more preferably 10°C to 40°C, for example, room temperature.

The present disclosure further provides a method for preparing the fused-piperidine compound of formula **I** or a pharmaceutically acceptable salt thereof, which includes Method **1** or Method **2:**
when R³ is independently hydrogen, halogen, or C₁-C₄ alkoxy, the method for preparing the fused-piperidine compound of formula **I** or the salt thereof is Method **1;**
Method **1** comprises the following steps: in the presence of a base, conducting a substitution reaction in a solvent between a compound of formula **II** and a compound of formula **III** to obtain the fused-piperidine compound of formula **I;** 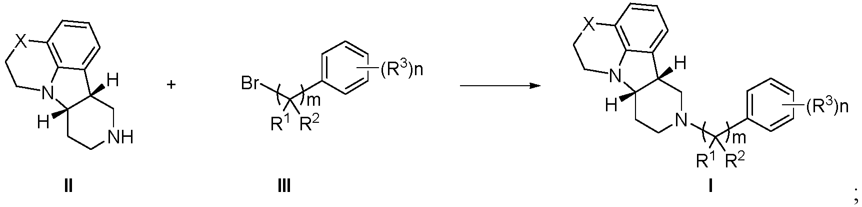
when R³ is independently hydroxyl, the method for preparing the fused-piperidine compound of formula **I** or the salt thereof is Method **2;**
Method **2** comprises the following steps: in the presence of BBr₃, reacting the fused-piperidine compound of formula **I** as described in method **1** in a solvent to obtain the fused-piperidine compound of formula **I** *(i.e.,* R³ is hydroxyl);
in Methods **1** and **2,** X, R^{a}, R^{b}, R^{c}, R¹, R², m, and n are the same as defined above.

In Method **1,** the conditions and procedures for the substitution reaction may be those conventional for such reactions in the art. The following conditions and procedures are particularly preferred in the present disclosure:
The base is preferably an organic base, for example, diisopropylethylamine (DIPEA). The molar ratio of the base to the compound of formula **II** is preferably (1 to 3):1, for example, 1:1.

The molar ratio of the compound of formula **III** to the compound of formula **II** is preferably (1 to 3):1, for example, 1.5:1.

The solvent is preferably a halogenated hydrocarbon organic solvent, for example, dichloromethane.

The temperature for the substitution reaction is preferably 50°C to 80°C, for example, 60°C.

In Method 2, the conditions and procedures for the reaction may be those conventional f in the art. The following conditions and procedures are particularly preferred in the present disclosure:

The solvent is preferably a halogenated hydrocarbon organic solvent, for example, dichloromethane.

The temperature for the substitution reaction is preferably 10°C to 40°C, for example, room temperature.

The present disclosure further provides a pharmaceutical composition comprising the fused-piperidine compound of formula **I'** or a pharmaceutically acceptable salt thereof, and a pharmaceutically acceptable excipient.

The present disclosure further provides a pharmaceutical composition comprising the fused-piperidine compound of formula **I** or a pharmaceutically acceptable salt thereof, and a pharmaceutically acceptable excipient.

The present disclosure further provides a use of a substance **A** in the preparation of a 5-HT_{2A} receptor agonist, wherein the substance A is the fused-piperidine compound of formula **I',** a pharmaceutically acceptable salt thereof, or the pharmaceutical composition.

The 5-HT_{2A} receptor agonist is preferably a 5-HT_{2A} receptor agonist of the downstream β-arrestin2 recruitment signaling pathway and/or a 5-HT_{2A} receptor agonist of the downstream Gq protein activation signaling pathway, for example, a 5-HT_{2A} receptor agonist of the downstream β-arrestin2 recruitment signaling pathway.

The present disclosure further provides a use of a substance **A** in the preparation of a 5-HT_{2A} receptor agonist, wherein the substance A is the fused-piperidine compound of formula **I,** a pharmaceutically acceptable salt thereof, or the pharmaceutical composition.

The 5-HT_{2A} receptor agonist is preferably a 5-HT_{2A} receptor agonist of the downstream β-arrestin2 recruitment signaling pathway and/or a 5-HT_{2A} receptor agonist of the downstream Gq protein activation signaling pathway, for example, a 5-HT_{2A} receptor agonist of the downstream β-arrestin2 recruitment signaling pathway.

The present disclosure further provides a use of a substance **A** in the preparation of a medicament;
the substance **A** is the fused-piperidine compound of formula **I',** a pharmaceutically acceptable salt thereof, or the pharmaceutical composition;
the medicament is a medicament for treating or preventing a disease associated with the 5-HT_{2A} receptor.

The disease associated with the 5-HT_{2A} receptor is preferably a disease associated with the downstream β-arrestin2 recruitment signaling pathway of the 5-HT_{2A} receptor and/or the downstream Gq protein activation signaling pathway of the 5-HT_{2A} receptor, preferably a disease associated with the downstream β-arrestin2 recruitment signaling pathway of the 5-HT_{2A} receptor, for example, depression.

The present disclosure further provides a use of a substance **A** in the preparation of a medicament;
the substance **A** is the fused-piperidine compound of formula **I,** a pharmaceutically acceptable salt thereof, or the pharmaceutical composition;
the medicament is a medicament for treating or preventing a disease associated with the 5-HT_{2A} receptor.

The disease associated with the 5-HT_{2A} receptor is preferably a disease associated with the downstream β-arrestin2 recruitment signaling pathway of the 5-HT_{2A} receptor and/or the downstream Gq protein activation signaling pathway of the 5-HT_{2A} receptor, preferably a disease associated with the downstream β-arrestin2 recruitment signaling pathway of the 5-HT_{2A} receptor, for example, depression.

The present disclosure further provides a use of a substance **A in** the preparation of a medicament for treating or preventing depression, wherein the substance **A** is the fused-piperidine compound of formula **I',** a pharmaceutically acceptable salt thereof, or the pharmaceutical composition.

The present disclosure further provides a use of a substance **A in** the preparation of a medicament for treating or preventing depression, wherein the substance **A** is the fused-piperidine compound of formula **I,** a pharmaceutically acceptable salt thereof, or the pharmaceutical composition.

The present disclosure further provides a method for treating or preventing depression, comprising administering to a subject (*e.g*., a human or a mouse) in need a therapeutically effective amount of substance A, wherein the substance A is the fused-piperidine compound of formula **I',** a pharmaceutically acceptable salt thereof, or the pharmaceutical composition.

In the present disclosure, room temperature refers to 10 to 30°C. Overnight refers to 8 to 15 hours.

In the present disclosure, the halogen is preferably fluorine, chlorine, bromine, or iodine.

In the present disclosure, the alkyl is preferably methyl, ethyl, *n*-propyl, isopropyl, *n-*butyl, isobutyl, or *tert*-butyl*.*

In the present disclosure, the alkoxy refers to an alkyl group, either cyclic or acyclic, connected through an oxygen bridge with the specified number of carbon atoms. Thus, the definition of alkoxy groups includes the definitions of both alkyl and cycloalkyl groups. In the present disclosure, the alkoxy is preferably C₁-C₄ alkoxy, more preferably methoxy, ethoxy, *n*-propoxy, isopropoxy, or *tert*-butoxy.

In the present disclosure, "ns" indicates no significant difference, "*" indicates P < 0.05, "**" indicates P < 0.01, "***" indicates P < 0.001, and "****" indicates P < 0.0001.

On the basis of not violating common knowledge in the art, the above preferred conditions can be arbitrarily combined to obtain the preferred examples of the present disclosure.

All reagents and raw materials used in the present disclosure are commercially available.

The positive progressive effect of the present disclosure is that: the fused-piperidine compound of the present disclosure has a superior inhibitory effect on depression and can effectively treat depression. Furthermore, the fused-piperidine compound of the present disclosure also avoids hallucinogenic side effects while treating depression.

### BRIEF DESCRIPTION OF THE DRAWINGS

Figure 1 shows a graph of the antidepressant effect of compound I-3 in the mouse tail suspension test from Biological Test Example 5, where "*" indicates P < 0.05 and "****" indicates P < 0.0001.
Figure 2 shows a graph of the antidepressant effect of compound I-3 in the mouse forced swim test from Biological Test Example 5, where "ns" indicates no significant difference and "****" indicates P < 0.0001.
Figure 3 shows a graph of the antidepressant effect of compound I-10 in the mouse tail suspension test from Biological Test Example 5, where "ns" indicates no significant difference and "****" indicates P < 0.0001.
Figure 4 shows a graph of the antidepressant effect of compound I-10 in the mouse forced swim test from Biological Test Example 5, where "ns" indicates no significant difference and "****" indicates P < 0.0001.
Figure 5 shows a graph of the head-twitch response in mice treated with compound I-3 in Biological Test Example 6.
Figure 6 shows a graph of the head-twitch response in mice treated with compound I-10 in Biological Test Example 6.
Figure 7 shows a graph of the head-twitch response in mice treated with compound I-23 in Biological Test Example 7.
Figure 8 shows a graph of the antidepressant effect of compound I-23 in the mouse tail suspension test at 1 day, 7 days, and 14 days after a single injection in Biological Test Example 7, where "ns" indicates no significant difference, "*" indicates P < 0.05, "**" indicates P < 0.01, "***" indicates P < 0.001, and "****" indicates P < 0.0001.

### DETAILED DESCRIPTION

### Example 1: Preparation of (6bR,10aS)-8-(2-methoxyphenyl)-3-methyl-2,3,6b,7,8,9,10,10a-octahydro-1H-pyrido[3',4':4,5]pyrrolo[1,2,3-de]quinoxaline (compound 1-1)

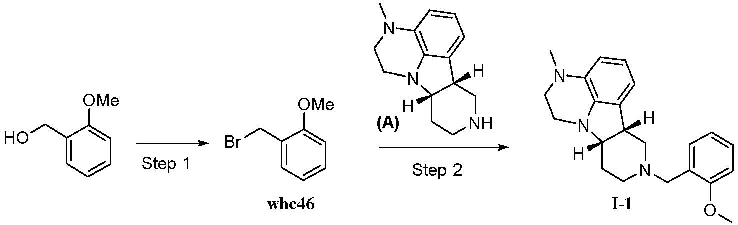

Step 1: To a round-bottom flask were added (2-methoxyphenyl)methanol (0.54 g, 3.91 mmol), CBr₄ (1.94 g, 5.87 mmol), and dichloromethane (20 mL). The mixture was then placed in an ice-water bath, and PPh₃ (1.54 g, 5.87 mmol) was added. The reaction was stirred at room temperature for 5 hours. After the reaction was completed, the mixture was concentrated under reduced pressure, and purified by flash silica gel column chromatography (eluent: 10 to 30% ethyl acetate/petroleum ether) to obtain **whc46** (0.75 g, yield of 96%) as a colorless oil. ¹H NMR (800 MHz, CDCl₃) δ 7.33 (d, *J =* 7.5 Hz, 1H), 7.31 - 7.27 (m, 1H), 6.92 (t, *J =* 7.4 Hz, 1H), 6.88 (d, *J* = 8.3 Hz, 1H), 4.57 (s, 2H), 3.90 (s, 3H).

Step 2: To a round-bottom flask were added starting material **(A)** ((6b*R*,10a*S*)-3-methyl-2,3,6b,7,8,9,10,10a-octahydro-1*H*-pyrido[3',4':4,5]pyrrolo[1,2,3-*de*]quinoxaline, CAS# 313368-85-3, purchased from Shanghai Bide Pharmatech Ltd.) (100 mg, 0.44 mmol), **whc46** (130 mg, 0.66 mmol), DMSO (5 mL), and diisopropylethylamine (DIPEA) (1 mL). The reaction was stirred at 60°C overnight. After the reaction was completed, added water and extracted with ethyl acetate. The organic phases were combined, washed once with brine, concentrated, and purified by flash silica gel column chromatography (eluent: 0 to 10% methanol/dichloromethane) to obtain **I-1** (100 mg, yield of 65%) as a yellow solid. ¹H NMR (800 MHz, CDCl₃) δ 7.50 (d, *J =* 7.2 Hz, 1H), 7.31 - 7.26 (m, 1H), 6.96 (t, *J =* 7.5 Hz, 1H), 6.87 (d, *J =* 8.3 Hz, 1H), 6.64 (t, *J =* 7.6 Hz, 1H), 6.48 (d, *J =* 7.4 Hz, 1H), 6.39 (d, *J =* 8.1 Hz, 1H), 3.80 - 3.78 (m, 5H), 3.60 - 3.56 (m, 1H), 3.47 - 3.43 (m, 1H), 3.30 - 3.22 (m, 3H), 3.10 (dd, *J* = 12.0, 6.6 Hz, 1H), 2.95 - 2.91 (m, 1H), 2.85 (s, 3H), 2.79 (t, *J =* 9.9 Hz, 1H), 2.61-2.56 (m, 1H), 2.27 - 2.23 (m, 2H), 1.98 (d, *J =* 14.4 Hz, 1H). ¹³C NMR (201 MHz, CDCl₃) δ 157.99, 137.70, 135.08, 131.85, 129.43, 129.02, 128.88, 120.71, 120.65, 112.74, 110.69, 109.08, 63.97, 55.51, 55.48, 55.11, 50.49, 48.16, 44.39, 40.58, 37.46, 23.81. HR-MS (ESI) *m*/*z* calculated for C₂₂H₂₈N₃O⁺ [M+H]⁺: 350.2227; found: 350.2233.

### Example 2: Preparation of 2-(((6bR,10aS)-3-methyl-2,3,6b,9,10,10a-hexahydro-1H-pyrido[3',4':4,5]pyrrolo[1,2,3-de]quinoxalin-8(7H)-yl)methyl)phenol (compound 1-2)

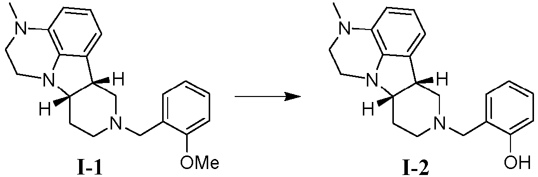

To a round-bottom flask were added **I-1** (47 mg, 0.13 mmol) and anhydrous dichloromethane (15 mL). Under an argon atmosphere, the mixture was cooled to -78°C, and BBr₃ (17% ether solution, 0.6 mL) was added. The reaction was stirred at room temperature for 3 hours. After the reaction was completed, saturated NaHCO₃ aqueous solution was added at low temperature (-30°C) to quench the reaction. The solvent was removed, and the residue was purified by flash silica gel column chromatography, followed by further purification by preparative liquid chromatography (20 to 80% MeOH/H₂O, t_{R} = 18.5 min) to obtain **I-2** (40 mg, 32%) as a yellow solid. ¹H NMR (800 MHz, CD₃OD) δ 7.34 - 7.29 (m, 2H), 6.97 - 6.89 (m, 2H), 6.68 - 6.64 (m, 1H), 6.53 - 6.47 (m, 2H), 4.28 (s, 2H), 3.56 (t, *J =* 10.9 Hz, 1H), 3.52 - 3.50 (m, 1H), 3.46 - 3.40 (m, 2H), 3.37 - 3.32 (m, 2H), 3.28 - 3.22 (m, 2H), 2.87 (s, 3H), 2.79 (t, *J* = 10.2 Hz, 1H), 2.74 (t, *J* = 12.4 Hz, 1H), 2.32 (d, *J =* 15.9 Hz, 1H), 2.16 - 2.10 (m, 1H). HR-MS (ESI) *m*/*z* calculated for C₂₁H₂₆N₃O⁺ [M+H]⁺: 336.2070; found: 336.2071.

### Example 3: Preparation of (6bR,10aS)-8-(2-methoxyphenyl)-3-methyl-2,3,6b,7,8,9,10,10a-octahydro-1H-pyrido[3',4':4,5]pyrrolo[1,2,3-de]quinoxaline (compound I-3)

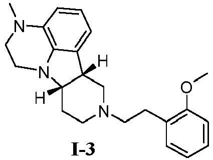

Following the method described in step 2 of Example 1, 1-(2-bromoethyl)-2-methoxybenzene (CAS# 36449-75-9) was reacted with starting material **(A)** to obtain compound **I-3** (220 mg, 90%) as a yellow oil. ¹H NMR (800 MHz, CDCl₃) δ 7.17 (t, *J* = 7.8 Hz, 1H), 7.13 (d, *J* = 7.4 Hz, 1H), 6.86 (t, *J =* 7.4 Hz, 1H), 6.83 - 6.80 (m, 1H), 6.66 (t, *J =* 7.7 Hz, 1H), 6.54 (d, *J =* 7.3 Hz, 1H), 6.41 (d, *J =* 7.9 Hz, 1H), 3.78 (s, 3H), 3.62 - 3.58 (m, 1H), 3.36 - 3.32 (m, 1H), 3.31- 3.28 (m, 1H), 3.27 -3.23 (m, 2H), 3.10 (dd, *J =* 11.7, 6.5 Hz, 1H), 2.93 - 2.89 (m, 3H), 2.86 (s, 3H), 2.82 (t, *J =* 10.0 Hz, 1H), 2.72 - 2.63 (m, 2H), 2.53 - 2.47 (m, 1H), 2.20 - 2.10 (m, 2H), 2.04 - 1.97 (m, 1H). ¹³C NMR (201 MHz, CDCl₃) δ 157.51, 137.87, 135.12, 130.39, 129.53, 127.85, 127.63, 120.59, 112.76, 110.38, 109.11, 64.31, 58.45, 55.61, 55.28, 50.64, 48.74, 44.44, 41.15, 37.55, 27.40, 24.37. HR-MS (ESI) *m*/*z* calculated for C₂₃H₃₀N₃O⁺ [M+H]⁺: 364.2383; found: 364.2386. Specific rotation: [α]_{D}²⁵ = -54° (c = 0.1, CHCl₃).

### Example 4: Preparation of (6bR,10aS)-8-(3-methoxyphenyl)-3-methyl-2,3,6b,7,8,9,10,10a-octahydro-1H-pyrido[3',4':4,5]pyrrolo[1,2,3-de]quinoxaline (compound 1-4)

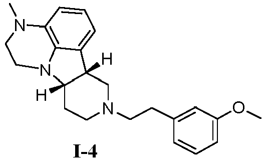

Following the method described in step 2 of Example 1, 1-(2-bromoethyl)-3-methoxybenzene (CAS# 2146-61-4) was reacted with starting material **(A)** to obtain compound **1-4** (80 mg, 84%) as a yellow oil. ¹H NMR (800 MHz, CDCl₃) δ 7.21 - 7.17 (m, 1H), 6.77 (d, *J =* 7.5 Hz, 1H), 6.76 - 6.72 (m, 2H), 6.66 (t, *J =* 7.6 Hz, 1H), 6.54 (d, *J =* 7.3 Hz, 1H), 6.42 (d, *J =* 8.0 Hz, 1H), 3.78 (s, 3H), 3.63 - 3.59 (m, 1H), 3.32 (dt, *J =* 10.1, 2.9 Hz, 1H), 3.29-3.22 (m, 2H), 3.22 - 3.18 (m, 1H), 2.99 - 2.95 (m, 1H), 2.87 (s, 3H), 2.84 (dd, *J =* 10.0, 3.0 Hz, 1H), 2.82 - 2.78 (m, 2H), 2.78 - 2.74 (m, 1H), 2.64 - 2.55 (m, 2H), 2.37 - 2.33 (m, 1H), 2.05 (t, *J* = 11.2 Hz, 1H), 2.00 - 1.93 (m, 2H). ¹³C NMR (201 MHz, CDCl₃) δ 159.70, 142.13, 138.03, 135.06, 130.04, 129.38, 121.16, 120.38, 114.54, 112.75, 111.37, 109.01, 64.58, 60.63, 56.30, 55.16, 50.71, 49.07, 44.41, 41.79, 37.61, 33.67, 25.06. HR-MS (ESI) *m*/*z* calculated for C₂₃H₃₀N₃O⁺ [M+H]⁺: 364.2383; found: 364.2385.

### Example 5: Preparation of (6bR,10aS)-8-(4-methoxyphenyl)-3-methyl-2,3,6b,7,8,9,10,10a-octahydro-1H-pyrido[3',4':4,5]pyrrolo[1,2,3-de]quinoxaline (compound I-5)

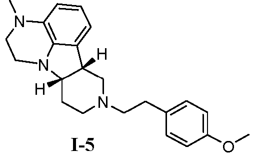

Following the method described in step 2 of Example 1, 1-(2-bromoethyl)-4-methoxybenzene (CAS# 14425-64-0) was reacted with starting material **(A)** to obtain compound **I-5** (40mg, 42%) as a yellow oil. ¹H NMR (800 MHz, CDCl₃) δ 7.13 - 7.08 (m, 2H), 6.84 - 6.81 (m, 2H), 6.66 (t, *J =* 7.6 Hz, 1H), 6.54 (d, *J =* 7.3 Hz, 1H), 6.42 (d, *J =* 7.9 Hz, 1H), 3.78 (s, 3H), 3.61 (td, *J =* 11.0, 10.5, 3.1 Hz, 1H), 3.32 (dt, *J =* 10.1, 3.0 Hz, 1H), 3.29-3.23 (m, 2H), 3.22 - 3.18 (m, 1H), 2.99 - 2.95 (m, 1H), 2.87 (s, 3H), 2.83 (td, *J =* 10.0, 2.9 Hz, 1H), 2.79 - 2.72 (m, 3H), 2.60 - 2.50 (m, 2H), 2.36 - 2.30 (m, 1H), 2.04 (t, *J =* 11.2 Hz, 1H), 1.99 - 1.94 (m, 2H). ¹³C NMR (201 MHz, CDCl₃) δ 157.97, 138.03, 135.05, 132.52, 130.06, 129.64, 120.37, 113.89, 112.76, 109.00, 64.59, 61.02, 56.34, 55.28, 50.71, 49.09, 44.41, 41.78, 37.60, 32.71, 25.05. HR-MS (ESI) *m*/*z* calculated for C₂₃H₃₀N₃O⁺ [M+H]⁺: 364.2383; found: 364.2388.

### Example 6: Preparation of (6bR,10aS)-8-(2,3-dimethoxyphenyl)-3-methyl-2,3,6b,7,8,9,10,10a-octahydro-1H-pyrido[3',4':4,5]pyrrolo[1,2,3-de]quinoxaline (compound I-6)

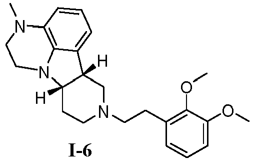

Following the method described in step 2 of Example 1, 1-(2-bromoethyl)-2,3-dimethoxybenzene (CAS# 709027-29-2) was reacted with starting material **(A)** to obtain compound **I-6** (130 mg, 75%) as a yellow oil. ¹H NMR (800 MHz, CDCl₃) δ 6.96 (t, *J* = 7.9 Hz, 1H), 6.78 - 6.75 (m, 2H), 6.65 (t, *J =* 7.6 Hz, 1H), 6.54 (d, *J =* 7.3 Hz, 1H), 6.41 (d, *J =* 7.9 Hz, 1H), 3.84 (s, 3H), 3.81 (s, 3H), 3.60 (td, *J =* 10.5, 3.0 Hz, 1H), 3.33 - 3.31 (m, 1H), 3.28 - 3.25 (m, 1H), 3.24 - 3.22 (m, 1H), 3.21 - 3.19 (m, 1H), 2.99 (dd, *J =* 11.6, 6.2 Hz, 1H), 2.86 (s, 3H), 2.84 (t, *J =* 8.2 Hz, 2H), 2.82 - 2.78 (m, 1H), 2.60 - 2.54 (m, 2H), 2.40 - 2.32 (m, 2H), 2.10 -2.03 (m, 1H), 1.99 - 1.97 (m, 2H). ¹³C NMR (201 MHz, CDCl₃) δ 152.80, 147.35, 138.07, 135.05, 134.21, 130.12, 123.88, 122.31, 120.34, 112.81, 110.53, 108.98, 64.63, 60.81, 59.76, 56.26, 55.73, 50.71, 48.98, 44.44, 41.78, 37.61, 27.62, 25.03. HR-MS (ESI) *m*/*z* calculated for C₂₄H₃₂N₃O₂⁺ [M+H]⁺: 394.2489; found: 394.2489.

### Example 7: Preparation of (6bR,10aS)-8-(2,4-methoxyphenyl)-3-methyl-2,3,6b,7,8,9,10,10a-octahydro-1H-pyrido[3',4':4,5]pyrrolo[1,2,3-de]quinoxaline (compound I-7)

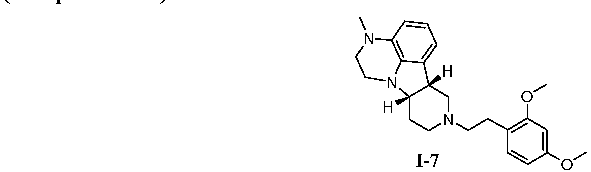

Following the method described in step 2 of Example 1, 1-(2-bromoethyl)-2,4-dimethoxybenzene (CAS# 37567-79-6) was reacted with starting material **(A)** to obtain compound 1-7 (72 mg, 84%) as a yellow oil. ¹H NMR (800 MHz, CDCl₃) δ 7.12 (d, *J* = 8.0 Hz, 1H), 6.71 (t, *J =* 7.7 Hz, 1H), 6.53 (d, *J =* 7.4 Hz, 1H), 6.44 (d, *J =* 8.0 Hz, 1H), 6.42 - 6.41 (m, 2H), 3.86 - 3.83 (m, 1H), 3.77 (s, 3H), 3.76 (s, 3H), 3.62 (t, *J =* 10.1 Hz, 1H), 3.50 (dd, *J* = 12.1, 6.5 Hz, 1H), 3.44 - 3.39 (m, 1H), 3.36 - 3.32 (m, 1H), 3.31 (d, *J =* 10.3 Hz, 2H), 3.17 - 3.11 (m, 2H), 3.11 - 3.05 (m, 3H), 2.88 (s, 3H), 2.86 - 2.83 (m, 1H), 2.81 - 2.74 (m, 1H), 2.61 - 2.59 (m, 1H), 2.19 - 2.14 (m, 1H). ¹³C NMR (201 MHz, CDCl₃) δ 160.33, 158.12, 137.07, 135.43, 131.05, 127.07, 121.49, 116.60, 112.53, 109.59, 104.43, 98.62, 62.94, 56.98, 55.37, 55.30, 53.37, 50.31, 47.85, 44.38, 38.77, 37.33, 24.75, 21.94. HR-MS (ESI) *m*/*z* calculated for C₂₄H₃₂N₃O₂⁺ [M+H]⁺: 394.2489; found: 394.2498.

### Example 8: Preparation of (6bR,10aS)-8-(2,5-dimethoxy)-3-methyl-2,3,6b,7,8,9,10,10a-octahydro-1H-pyrido[3',4':4,5]pyrrolo[1,2,3-de]quinoxaline (compound I-8)

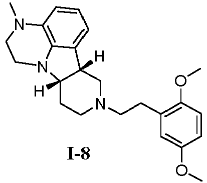

Following the method described in step 2 of Example 1, 1-(2-bromoethyl)-2,5-dimethoxybenzene (CAS# 99187-42-5) was reacted with starting material (A) to obtain compound 1-8 (50 mg, 13%) as a yellow oil. ¹H NMR (800 MHz, CDCl₃) δ 6.75 (d, *J =* 8.8 Hz, 1H), 6.73 (d, *J =* 3.1 Hz, 1H), 6.69 (dd, *J =* 8.8, 3.1 Hz, 1H), 6.66 (t, *J =* 7.7 Hz, 1H), 6.54 (d, *J =* 7.3 Hz, 1H), 6.41 (d, *J =* 7.9 Hz, 1H), 3.75 (s, 3H), 3.74 (s, 3H), 3.60 (td, *J =* 10.7, 3.0 Hz, 1H), 3.31 (dt, *J =* 10.2, 3.0 Hz, 1H), 3.29 - 3.22 (m, 3H), 3.08 - 3.03 (m, 1H), 2.86 (s, 3H), 2.85 - 2.81 (m, 4H), 2.66 - 2.64 (m, 2H), 2.44 (t, *J =* 12.0 Hz, 1H), 2.12 (t, *J =* 11.3 Hz, 1H), 2.09 - 2.02 (m, 1H), 2.00 - 1.97 (m, 1H). ¹³C NMR (201 MHz, CDCl₃) δ 153.49, 151.78, 137.92, 135.05, 129.76, 129.46, 120.43, 116.63, 112.74, 111.48, 111.32, 109.01, 64.40, 58.56, 55.92, 55.77, 55.68, 50.63, 48.75, 44.39, 41.35, 37.54, 27.70, 24.58. HR-MS (ESI) *m*/*z* calculated for C₂₄H₃₂N₃O₂⁺ [M+H]⁺: 394.2489; found: 394.2489.

### Example 9: Preparation of 2-(2-((6bR,10aS)-3-methyl-2,3,6b,9,10,10a-hexahydro-1H-pyrido[3',4':4,5]pyrrolo[1,2,3-de]quinoxalin-8(7H)-yl)ethyl)phenol (compound 1-9)

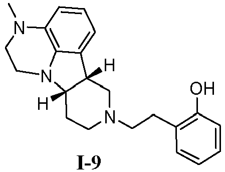

Following the method described in Example 2, **I-3** was subjected to a demethylation reaction to obtain compound **I-9** (100 mg, 70%) as a yellow solid. ¹H NMR (800 MHz, CD₃OD) δ 6.98 (t, *J =* 7.7 Hz, 1H), 6.95 (d, *J =* 7.5 Hz, 1H), 6.71 (d, *J =* 8.0 Hz, 1H), 6.65 (t, *J =* 7.4 Hz, 1H), 6.54 (t, *J =* 7.6 Hz, 1H), 6.44 (d, *J =* 7.3 Hz, 1H), 6.36 (d, *J =* 7.9 Hz, 1H), 3.48 - 3.43 (m, 1H), 3.24 - 3.21 (m, 2H), 3.14 - 3.10 (m, 2H), 2.98 - 2.96 (m, 1H), 2.86 - 2.81 (m, 1H), 2.78 (s, 3H), 2.77 - 2.76 (m, 2H), 2.72 - 2.67 (m, 1H), 2.60 - 2.57 (m, 1H), 2.55-2.51 (m, 1H), 2.40 (t, *J =* 12.2 Hz, 1H), 2.05 - 1.98 (m, 2H), 1.96 - 1.92 (m, 1H). ¹³C NMR (201 MHz, CD₃OD) δ 157.45, 139.31, 136.51, 131.74, 130.77, 128.82, 128.79, 121.76, 120.44, 117.55, 114.08, 110.78, 65.79, 60.28, 57.16, 51.86, 49.93, 45.67, 42.65, 38.06, 31.08, 25.37. HR-MS (ESI) *m*/*z* calculated for C₂₂H₂₈N₃O⁺ [M+H]⁺: 350.2227; found: 350.2227.

### Example 10: Preparation of (6bR,10aS)-8-(3-(2-methoxy)propyl)-3-methyl-2,3,6b,7,8,9,10,10a-octahydro-1H-pyrido[3',4':4,5]pyrrolo[1,2,3-de]quinoxaline (compound I-10)

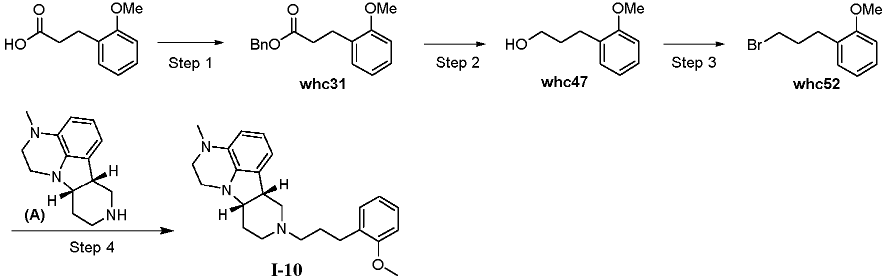

Step 1: To a round-bottom flask were added 3-(2-methoxyphenyl)propanoic acid (2.0 g, 11.1 mmol), DMF (10 mL), and Cs₂CO₃ (10.8 g, 33.3 mmol). BnBr (2.85 g, 16.7 mmol) was then added. The reaction was stirred at room temperature overnight. After the reaction was completed, the solvent was removed. The residue was purified by flash silica gel column chromatography (eluent: 20 to 30% ethyl acetate/petroleum ether) to obtain **whc31** (2.8 g, yield of 97%) as a colorless oil. ¹H NMR (800 MHz, CDCl₃) δ 7.36 - 7.34 (m, 2H), 7.33 - 7.29 (m, 3H), 7.21 - 7.18 (m, 1H), 7.12 (dd, *J =* 7.4, 1.7 Hz, 1H), 6.86 (dd, *J =* 7.4, 1.1 Hz, 1H), 6.85 - 6.82 (m, 2H), 5.11 (s, 2H), 3.80 (s, 3H), 2.98 - 2.95 (m, 2H), 2.69 - 2.65 (m, 2H).

Step 2: To a dry round-bottom flask was added **whc31** (benzyl 3-(2-methoxyphenyl)propanoate, 1.0 g, 3.7 mmol), which was dissolved in THF (15 mL). The mixture was stirred in a low-temperature reactor at -10°C under an argon atmosphere. After 10 minutes, DABAL-H (23 mL, 1 M in hexane) was added. The reaction was then stirred at 0°C for 5 hours. After the reaction was completed, saturated potassium sodium tartrate solution (5 mL) was added at low temperature (-10°C) to quench the reaction. After the solvent was removed, the residue was purified by flash silica gel column chromatography (eluent: 20 to 50% ethyl acetate/petroleum ether) to obtain **whc47** (0.50 g, yield of 81%) as a colorless oil. ¹H NMR (800 MHz, CDCl₃) δ 7.19 (t, *J =* 7.8 Hz, 1H), 7.14 (d, *J =* 7.5 Hz, 1H), 6.90 (t, *J =* 7.4 Hz, 1H), 6.86 (d, *J =* 8.2 Hz, 1H), 3.83 (s, 3H), 3.60 (t, *J =* 6.2 Hz, 2H), 2.72 (t, *J =* 7.4 Hz, 2H), 1.88 - 1.82 (m, 2H).

Step 3: Following the method described in step 1 of Example 1, **whc47** was subjected to a bromination reaction to obtain compound **whc52** (0.62 g, yield of 90%) as a colorless oil. ¹H NMR (800 MHz, CDCl₃) δ 7.22 - 7.17 (m, 1H), 7.15 (dd, *J =* 7.4, 1.7 Hz, 1H), 6.91 - 6.87 (td, *J =* 7.4, 1.1 Hz, 1H), 6.85 (dd, *J =* 8.1, 1.1 Hz, 1H), 3.82 (s, 3H), 3.40 (t, *J =* 6.8 Hz, 2H), 2.78 - 2.74 (m, 2H), 2.18 - 212 (m, 2H).

Step 4: Following the method described in step 2 of Example 1, **whc52** (1-(3-bromopropyl)-2-methoxybenzene) was reacted with starting material (A) to obtain compound **I-10** (80 mg, 96%) as a yellow oil. ¹H NMR (800 MHz, CD₃OD) δ 7.24 - 7.18 (m, 1H), 7.13 (d, *J =* 7.3 Hz, 1H), 6.93 (d, *J =* 8.2 Hz, 1H), 6.87 (t, *J =* 7.4 Hz, 1H), 6.67 (d, *J =* 9.7 Hz, 1H), 6.59 (d, *J =* 7.3 Hz, 1H), 6.53 (d, *J =* 8.0 Hz, 1H), 3.82 (s, 3H), 3.57 - 3.53 (m, 2H), 3.47-3.44 (m, 1H), 3.43 - 3.40 (m, 1H), 3.39 - 3.32 (m, 2H), 3.29 - 3.25 (m, 1H), 3.19 - 3.15 (m, 1H), 3.13 - 3.04 (m, 2H), 2.88 (s, 3H), 2.84 - 2.80 (m, 1H), 2.69 (t, *J =* 7.5 Hz, 2H), 2.65 (t, *J* = 12.3 Hz, 1H), 2.36 - 2.31 (m, 1H), 2.16 - 2.09 (m, 1H), 2.07 - 1.99 (m, 2H). ¹³C NMR (201 MHz, CDCl₃) δ 157.02, 137.25, 134.89, 129.50, 128.97, 128.28, 127.07, 120.46, 120.11, 112.22, 110.02, 108.89, 63.48, 57.39, 54.94, 54.52, 50.15, 48.05, 43.99, 39.96, 37.13, 27.50, 25.02, 23.11. HR-MS (ESI) *m*/*z* calculated for C₂₄H₃₂N₃O⁺ [M+H]⁺: 378.2540; found: 378.2547. Specific rotation: [α]_{D}²⁵ = -41° (c = 0.1, CHCl₃).

### Example 11: Preparation of 2-(3-((6bR,10aS)-3-methyl-2,3,6b,9,10,10a-hexahydro-1H-pyrido[3',4':4,5]pyrrolo[1,2,3-de]quinoxalin-8(7H)-yl)propyl)phenol (compound I-11)

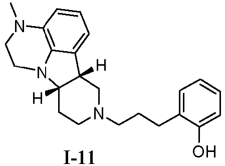

Following the method described in Example 2, **I-10** was subjected to a demethylation reaction to obtain compound **I-11** (20 mg, 34%) as a gray solid. ¹H NMR (800 MHz, CD₃OD) δ 7.51 (s, 1H), 7.10 (dd, *J =* 7.4, 1.7 Hz, 1H), 7.05 (td, *J =* 7.7, 1.7 Hz, 1H), 6.79 - 6.75 (m, 2H), 6.69 (d, *J =* 7.8 Hz, 1H), 6.62 - 6.58 (m, 1H), 6.54 (d, *J =* 8.0 Hz, 1H), 3.60 - 3.56 (m, 2H), 3.53 - 3.45 (m, 1H), 3.45 - 3.41 (m, 1H), 3.40 - 3.35 (m, 1H), 3.30 - 3.26 (m, 1H), 3.18 (t, *J =* 13.0 Hz, 1H), 3.14 - 3.10 (m, 2H), 2.89 (s, 3H), 2.85 - 2.81 (m, 1H), 2.70 (t, *J =* 7.5 Hz, 2H), 2.66 (t, *J =* 12.3 Hz, 1H), 2.35 (d, *J =* 16.3 Hz, 1H), 2.19 - 2.13 (m, 1H), 2.10 - 2.06 (m, 3H). ¹³C NMR (201 MHz, CD₃OD) δ 153.62, 136.36, 133.00, 128.48, 126.02, 124.86, 119.73, 118.03, 113.29, 111.74, 109.11, 61.41, 55.44, 52.00, 49.00, 42.49, 37.94, 35.47, 27.55, 25.44, 22.75, 20.56, 18.11, 11.71. HR-MS (ESI) *m*/*z* calculated for C₂₃H₃₀N₃O⁺ [M+H]⁺: 364.2383; found: 364.2386.

### Examples 12 and 13: Preparation of (8aS,12aR)-11-(2-methoxyphenethyl)-4-methyl-4,5,6,7,8a,9,10,11,12,12a-decahydro-[1,4]diazepino[3,2,1-hi]pyrido[4,3-b]indole (compound I-12) and (8aR,12aS)-11-(2-methoxyphenethyl)-4-methyl-4,5,6,7,8a,9,10,11,12,12a-decahydro-[1,4]diazepino[3,2,1-hi]pyrido[4,3-b]indole (compound I-13)

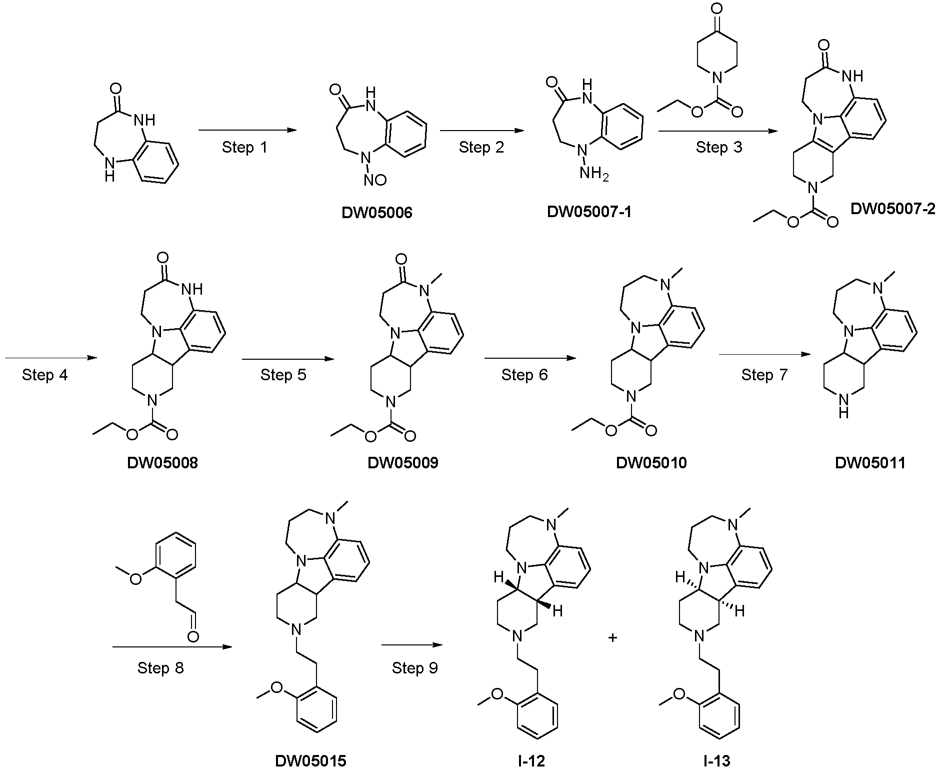

Step 1: To a round-bottom flask was added 1,3,4,5-tetrahydro-2*H*-1,5-benzodiazepin-2-one (1.6 g, 10 mmol), which was dissolved in a mixture of acetic acid and water (2:1, 15 mL) with stirring. The mixture was then cooled to 0°C. A solution of sodium nitrite (770 mg, 11 mmol) in water (5 mL) was slowly added dropwise to the reaction mixture. After the dropwise addition was completed, the reaction system was stirred at room temperature for 2 hours. After the reaction was completed, the mixture was filtered to obtain **DW05006** (1.7 g, yield of 89%) as a pale yellow solid. ¹H NMR (800 MHz, DMSO-d₆) δ 10.06 (s, 1H), 7.54 (dd, *J* = 7.9, 1.3 Hz, 1H), 7.50 (td, *J = 7.8,* 1.4 Hz, 1H), 7.31 (td, *J* = 7.7, 1.4 Hz, 1H), 7.21 (dd, *J =* 8.0, 1.2 Hz, 1H), 4.14 - 4.08 (m, 2H), 2.65 (dd, *J =* 7.5, 5.4 Hz, 2H). HRMS (ESI) *m*/*z* calculated for C₉H₁₀N₃O₂⁺ [M+H]⁺: 192.0768, found: 192.0771.

Step 2: To a round-bottom flask was added **DW05006** (0.96 g, 5 mmol), which was dissolved in acetic acid (10 mL) at room temperature with stirring. Subsequently, the reaction mixture was placed in an ice bath, and zinc powder (1.6 g, 25 mmol) was slowly added to the reaction system in batches. After the addition was completed, the reaction system was stirred at room temperature for 1 hour, and the reaction was completed.

Step 3: The insoluble material was filtered off from the reaction mixture of the previous step. To the filtrate were added with *N*-ethoxycarbonyl-4-piperidone (0.75 mL, 5 mmol) and 1 N hydrochloric acid aqueous solution (0.3 mL). The reaction system was heated to reflux. After 2 hours, the reaction was completed. The reaction mixture was cooled to room temperature and distilled under reduced pressure to remove most of the acetic acid. Cold ethanol was then slowly added, resulting in the precipitation of a solid. The mixture was filtered to obtain **DW05007-2** (685 mg, yield of 44%) as a pale yellow solid. ¹H NMR (800 MHz, DMSO-d₆) δ 10.10 (s, 1H), 7.13 (d, *J =* 7.6 Hz, 1H), 6.93 (t, *J =* 7.7 Hz, 1H), 6.77 (d, *J* = 7.3 Hz, 1H), 4.57 (s, 2H), 4.25 - 4.17 (m, 2H), 4.09 (q, *J =* 7.1 Hz, 2H), 3.77 (t, *J =* 5.7 Hz, 2H), 2.91 - 2.84 (m, 2H), 2.79 (t, *J =* 5.6 Hz, 2H), 1.25 - 1.17 (m, 3H). HRMS (ESI) *m*/*z* calculated for C₁₇H₂₀N₃O₃⁺ [M+H]⁺: 314.1499, found: 314.1521.

Step 4: To a round-bottom flask was added **DW05007-2** (626 mg, 2 mmol), which was dissolved in trifluoroacetic acid (10 mL) at 0°C with stirring. Sodium cyanoborohydride (151 mg, 2.4 mmol) was slowly added to the reaction mixture in batches. After the addition was completed, the reaction system was stirred and reacted at room temperature for 1 hour. After the reaction was completed, 10 mL of water was added to quench the reaction. The reaction mixture was distilled under reduced pressure to remove most of the trifluoroacetic acid, and the pH of the mixture was adjusted to 9.0 using 2 N sodium hydroxide aqueous solution. The mixture was extracted three times with ethyl acetate, and the organic phases were combined, washed with brine, and dried over anhydrous sodium sulfate. The organic phase was concentrated and then purified by flash silica gel column chromatography (eluent: 0 to 50% petroleum ether/ethyl acetate) to obtain **DW05008** (441 mg, yield of 70%) as a white solid. ¹H NMR (800 MHz, CDCl₃) δ 7.84 (s, 1H), 6.88 (d, *J =* 5.6 Hz, 1H), 6.70 (t, *J =* 7.4 Hz, 1H), 6.63 (d, *J =* 7.9 Hz, 1H), 4.18 - 4.09 (m, 2H), 3.93 & 3.80 (1H), 3.64 - 3.59 (m, 2H), 3.51-3.45 (m, 1H), 3.34 - 3.30 (m, 2H), 3.24 - 3.21 (m, 1H), 3.20 - 3.07 (m, 1H), 2.91 - 2.83 (m, 2H), 1.91 (s, 2H), 1.26 (t, *J =* 6.8 Hz, 3H). HRMS (ESI) *m*/*z* calculated for C₁₇H₂₂N₃O₃⁺ [M+H]⁺: 316.1656, found: 316.1670.

Step 5: To a round-bottom flask were added **DW05008** (315 mg, 1 mmol) and iodomethane (0.1 mL, 1.2 mmol). The mixture was dissolved in anhydrous *N,N-*dimethylformamide (10 mL) at room temperature with stirring. 60% sodium hydride (45 mg, 1.1 mmol) was slowly added in batches, and the mixture was stirred and reacted at room temperature for 2 hours. After the reaction was completed, 10 mL of water was added to quench the reaction. The mixture was extracted three times with ethyl acetate, and the organic phases were combined, washed with saturated brine, and dried over anhydrous sodium sulfate. The organic phase was concentrated and then purified by flash silica gel column chromatography (eluent: 0 to 50% petroleum ether/ethyl acetate) to obtain **DW05009** (300 mg, yield of 91%) as a colorless oil. ¹H NMR (800 MHz, CDCl₃) δ 6.94 - 6.92 (m, 2H), 6.76 (t, *J =* 7.0 Hz, 1H), 4.17 - 4.07 (m, 2H), 3.82 (t, *J =* 11.6 Hz, 1H), 3.65 - 3.58 (m, 2H), 3.52-3.45 (m, 1H), 3.42 - 3.36 (m, 1H), 3.35 (s, 3H), 3.33 - 3.26 (m, 1H), 3.04 - 3.01 (m, 1H), 2.70 (t, *J =* 12.4 Hz, 1H), 2.55 (dd, *J =* 13.4, 4.7 Hz, 1H), 1.98 - 1.86 (m, 2H), 1.76 (s, 1H), 1.24 (t, *J =* 7.0 Hz, 3H). HRMS (ESI) *m*/*z* calculated for C₁₈H₂₄N₃O₃⁺ [M+H]⁺: 330.1812, found: 330.1833.

Step 6: To a round-bottom flask was added **DW05009** (329 mg, 1 mmol), which was dissolved in anhydrous tetrahydrofuran (10 mL). The reaction system was placed in an ice bath, and a solution of borane in tetrahydrofuran (1 M, 2 mL) was added dropwise thereto. After the addition was completed, the reaction system was heated to reflux for 1 hour. After the reaction was completed, the reaction was cooled to room temperature. 2 N hydrochloric acid aqueous solution (1 mL) was added, and the mixture continued to be heated to reflux and stirred. After 30 minutes, the reaction was placed at room temperature, and the pH of the mixture was adjusted to 9.0 using 4 N sodium hydroxide aqueous solution. The mixture was extracted three times with ethyl acetate, and the organic phases were combined, washed with saturated brine, and dried over anhydrous sodium sulfate. The organic phase was concentrated and then purified by flash silica gel column chromatography (eluent: 0 to 40% petroleum ether/ethyl acetate) to obtain **DW05010** (300 mg, yield of 69%) as a colorless oil. HRMS (ESI) *m*/*z* calculated for C₁₈H₂₆N₃O₂⁺ [M+H]⁺: 316.2020, found: 316.2031.

Step 7: **DW05010** (315 mg, 1 mmol) was dissolved in n-butanol (10 mL), and potassium hydroxide (672 mg, 12 mmol) was added. The reaction was heated to 120°C, and refluxed with stirring for 5 hours. After the reaction was completed, the mixture was cooled to room temperature. 10 mL of water was added and the mixture was extracted three times with ethyl acetate. The organic phases were combined, washed with saturated brine, and dried over anhydrous sodium sulfate. The organic phase was concentrated and then purified by flash silica gel column chromatography (eluent: 0 to 10% methanol/dichloromethane) to obtain **DW05011** (137 mg, yield of 56%) as a pale yellow solid. ¹H NMR (800 MHz, CDCl₃) δ 6.75 (t, *J* = 7.6 Hz, 1H), 6.66 (d, *J = 7.1* Hz, 1H), 6.61 (d, *J* = 8.0 Hz, 1H), 3.50 - 3.45 (m, 1H), 3.29 - 3.22 (m, 2H), 3.14 (dt, *J =* 10.3, 6.6 Hz, 1H), 3.05 - 3.02 (m, 1H), 2.94 (td, *J =* 12.1, 3.0 Hz, 1H), 2.90 (s, 3H), 2.89 - 2.88 (m, 1H), 2.87 - 2.84 (m, 1H), 2.65 (td, *J =* 11.7, 3.5 Hz, 1H), 2.52 (dd, *J* = 12.6, 10.4 Hz, 1H), 2.02 - 1.97 (m, 1H), 1.97 - 1.94 (m, 1H), 1.92 - 1.89 (m, 1H), 1.84 - 1.80 (m, 1H). HRMS (ESI) *m*/*z* calculated for C₁₅H₂₂N₃⁺ [M+H]⁺: 244.1808, found: 244.1813.

Step 8: To a round-bottom flask were added **DW05011** (243 mg, 1 mmol) and 4-methoxyphenylacetaldehyde (225 mg, 1.5 mmol). The mixture was dissolved in methanol (10 mL). Sodium cyanoborohydride (94 mg, 1.5 mmol) was slowly added in batches. After the addition was completed, the mixture continued to be stirred at room temperature overnight. After the reaction was completed, 10 mL of water was added to quench the reaction. The mixture was extracted three times with ethyl acetate, and the organic phases were combined, washed with saturated brine, and dried over anhydrous sodium sulfate. The organic phase was concentrated and then purified by flash silica gel column chromatography (eluent: 0 to 10% methanol/dichloromethane) to obtain **DW05015** (302 mg, yield of 80%) as a pale yellow oil. ¹H NMR (800 MHz, CDCl₃) δ 7.23 (td, *J =* 8.2, 1.5 Hz, 1H), 7.16 (d, *J =* 7.4 Hz, 1H), 6.89 (t, *J =* 7.4 Hz, 1H), 6.84 (d, *J =* 8.2 Hz, 1H), 6.81 (t, *J =* 7.7 Hz, 1H), 6.71 (d, *J =* 7.1 Hz, 1H), 6.67 (d, *J =* 8.1 Hz, 1H), 3.81 (s, 3H), 3.59 - 3.56 (m, 1H), 3.52 - 3.47 (m, 1H), 3.29 - 3.27 (m, 1H), 3.27 - 3.23 (m, 1H), 3.23 - 3.17 (m, 2H), 3.00 (t, *J =* 8.1 Hz, 2H), 2.96 - 2.84 (m, 4H), 2.92 (s, 3H), 2.82 - 2.78 (m, 1H), 2.63 (td, *J =* 12.0, 3.0 Hz, 1H), 2.36 - 2.34 (m, 1H), 2.29 - 2.26 (t, *J =* 10.5 Hz, 1H), 2.19 - 2.17 (m, 1H), 2.06 - 2.02 (m, 1H), 1.91 - 1.87 (m, 1H). ¹³C NMR (201 MHz, CDCl₃) δ 157.44, 142.97, 140.15, 130.71, 128.66, 121.61, 121.00, 115.85, 115.56, 110.57, 62.71, 60.54, 57.60, 55.44, 55.12, 49.97, 48.66, 42.43, 39.25, 29.64, 26.55, 23.34. HRMS (ESI) *m*/*z* calculated for C₂₄H₃₂N₃O⁺ [M+H]⁺: 378.2540, found: 378.2559.

Chiral analysis conditions: instrument: UPCC (Waters); chiral column: CHIRALPAK OD (Daicel); column volume: 4.6 × 100 mm (5 µm packing); temperature: 40°C; mobile phase: CO₂/MeOH [0.2% ammonia methanol solution (7 M)] = 70/30; flow rate: 3.0 mL/min; detection wavelength: 214 nm.

Chiral preparation conditions: instrument: SFC-150 (Waters); chiral column: OD (Daicel); column volume: 20 × 250 mm (10 µm packing); temperature: 35°C; mobile phase: CO₂/MeOH [0.2% ammonia methanol solution (7 M)] = 60/40; flow rate: 100.0 mL/min; detection wavelength: 214 nm. **I-12** (HRMS (ESI) *m*/*z* calculated for C₂₄H₃₂N₃O⁺ [M+H]⁺: 378.2540, found: 378.2538; e.e. 99.64%; specific rotation: [α]_{D}²⁵ = -65° (c = 0.1, CHCl₃), the optical rotation direction is consistent with that of compound I-3, being levorotatory, indicating an identical chiral configuration) and **I-13** (HRMS (ESI) *m*/*z* calculated for C₂₄H₃₂N₃O⁺ [M+H]⁺: 378.2540, found: 378.2557; e.e. 99.44%; specific rotation: [α]_{D}²⁵ = +103° (c = 0.1, CHCl₃), the optical rotation direction is opposite to that of compound I-3, being dextrorotatory, indicating an opposite chiral configuration) were obtained.

### Examples 14 and 15: Preparation of (8aS,12aR)-11-(3-(2-methoxyphenethyl)propyl)-4-methyl-4,5,6,7,8a,9,10,11,12,12a-decahydro-[1,4]diazepino[3,2,1-hi]pyrido[4,3-b]indole (compound I-14) and (8aR,12aS)-11-(3-(2-methoxyphenethyl)propyl)-4-methyl-4,5,6,7,8a,9,10,11,12,12a-decahydro-[1,4]diazepino[3,2,1-hi]pyrido[4,3-b]indole (compound I-15)

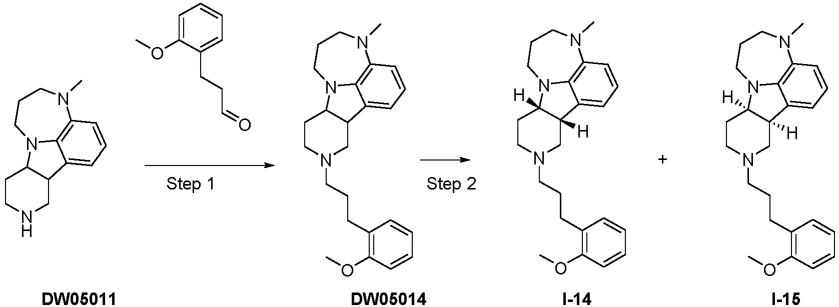

Step 1: Following the method described in step 8 of Examples **12** and **13,** 4-methoxyphenylacetaldehyde was replaced with 4-methoxyphenylpropionaldehyde to obtain **DW05014** (278 mg, yield of 81%) as a pale yellow oil. ¹H NMR (800 MHz, CDCl₃) δ 7.19 (t, *J =* 7.8 Hz, 1H), 7.07 (d, *J =* 7.3 Hz, 1H), 6.87 (t, *J =* 7.3 Hz, 1H), 6.83 (d, *J =* 8.2 Hz, 1H), 6.80 (t, *J =* 7.6 Hz, 1H), 6.66 - 6.64 (m, 2H), 3.81 (s, 3H), 3.66 - 3.63 (m, 1H), 3.48 (t, *J =* 9.3 Hz, 1H), 3.26 - 3.24 (m, 2H), 3.19 - 3.15 (m, 1H), 2.96 - 2.94 (m, 1H), 2.90 (s, 3H), 2.84-2.76 (m, 3H), 2.68 - 2.65 (m, 2H), 2.64 - 2.62 (m, 1H), 2.47 - 2.43 (m, 1H), 2.27 (t, *J =* 11.3 Hz, 1H), 2.17 (d, *J* = 15.8 Hz, 1H), 2.07 - 2.05 (m, 2H), 2.04 - 2.02 (m, 2H), 1.88 - 1.84 (m, 1H). ¹³C NMR (201 MHz, CDCl₃) δ 157.32, 142.65, 140.14, 129.99, 127.92, 121.62, 120.62, 115.83, 115.36, 110.46, 62.24, 57.31, 55.34, 54.90, 54.56, 49.74, 48.30, 42.21, 38.64, 29.40, 27.59, 24.13, 22.76. HRMS (ESI) *m*/*z* calculated for C₂₅H₃₄N₃O⁺ [M+H]⁺: 392.2696, found: 392.2712.

Step 2: **DW05014** was subjected to chiral resolution.

Chiral analysis method: instrument: UPCC (Waters); chiral column: CHIRALPAK IG (Daicel); column volume: 4.6 × 100 mm (5 µm packing); temperature: 40°C; mobile phase: CO₂/MeOH [0.2% ammonia methanol solution (7 M)] = 70/30; flow rate: 3.0 mL/min; detection wavelength: 214 nm.

Chiral preparation method: instrument: SFC-150 (Waters); chiral column: IG (Daicel); column volume: 20 × 250 mm (10 µm packing); temperature: 35°C; mobile phase: CO₂/MeOH [0.2% ammonia methanol solution (7 M)] = 50/50; flow rate: 100.0 mL/min; detection wavelength: 214 nm **I-14** (HRMS (ESI) *m*/*z* calculated for C₂₅H₃₄N₃O⁺ [M+H]⁺: 392.2697, found: 392.2712; e.e. 99.68%; specific rotation: [α]_{D}²⁵ = -55° (c = 0.1, CHCl₃), the optical rotation direction is consistent with that of compound **I-10,** being levorotatory, indicating an identical chiral configuration) and **I-15** (HRMS (ESI) *m*/*z* calculated for C₂₅H₃₄N₃O⁺ [M+H]⁺: 392.2696, found: 392.2709; e.e. 100%; specific rotation: [α]_{D}²⁵ = +95° (c = 0.1, CHCl₃), the optical rotation direction is opposite to that of compound **I-10,** being dextrorotatory, indicating an opposite chiral configuration) were obtained.

### Example 16: Preparation of (7aS,11aR)-10-(2-methoxyphenethyl)-5,6,7a,8,9,10,11,11a-octahydro-4H-pyrido[3',4':4,5]pyrrolo[3,2,1-ij]quinoline (compound I-16)

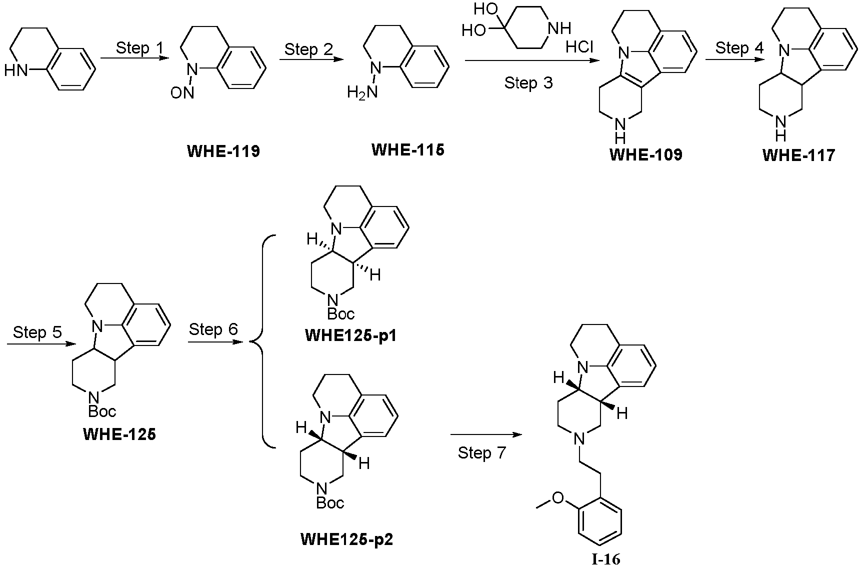

Step 1: Tetrahydroquinoline (4 g, 30 mmol) was dissolved in a mixed solvent of glacial acetic acid (40 mL) and water (10 mL). A solution of NaNO₂ (2.28 g, 33 mmol) in water (5 mL) was then added dropwise. The mixture was stirred at room temperature overnight. After the reaction was completed, the solvent was removed under reduced pressure. The concentrate was dissolved in ethyl acetate and washed once with saturated NaHCO₃ aqueous solution. The organic phase was concentrated and purified by flash silica gel column chromatography (eluent: 0 to 10% ethyl acetate/petroleum ether) to obtain **WHE-119** (4.5 g, yield of 92%) as an orange-yellow oil. ¹H NMR (800 MHz, CDCl₃) δ 8.06 (d, *J* = 8.2 Hz, 1H), 7.31 - 7.25 (m, 1H), 7.24 - 7.19 (m, 2H), 3.92 - 3.88 (m, 2H), 2.82 - 2.79 (m, 2H), 2.01 (p, *J =* 6.1 Hz, 2H). HR-MS (ESI) *m*/*z* calculated for C₉H₁₁N₂O⁺ [M+H]⁺: 163.0866, found: 163.0873.

Step 2: Under an argon atmosphere, LiAlH₄ (485 mg, 12.8 mmol) was suspended in anhydrous THF (25 mL). The mixture was cooled in an ice-water bath, and a solution of the starting material **WHE-119** (1.3 g, 7.98 mmol) in THF (5 mL) was added. The reaction mixture was stirred in an ice-water bath for 30 minutes, then slowly warmed to room temperature, and stirred overnight. After the reaction was completed, potassium sodium tartrate solution was added to quench the reaction, then added water, and filtered. The filtrate was extracted three times with ethyl acetate, and the organic phases were combined. The organic phase was concentrated and purified by flash silica gel column chromatography (eluent: 10 to 30% ethyl acetate/petroleum ether) to obtain **WHE-115** (0.95 g, yield of 81%) as a yellow solid. ¹H NMR (600 MHz, CDCl₃) δ 7.15 (d, *J* = 8.2, 1.4 Hz, 1H), 7.14 - 7.09 (m, 1H), 6.96 (d, *J* = 7.4 Hz, 1H), 3.34 - 3.30 (m, 2H), 2.76 (t, *J* = 6.6 Hz, 2H), 2.09 - 2.02 (m, 2H). HR-MS (ESI) *m*/*z* calculated for C₉H₁₃N₂⁺ [M+H]⁺: 149.1073, found: 149.1075.

Step 3: **WHE-115** (620 mg, 4.19 mmol) and 4,4-dihydroxypiperidine hydrochloride (650 mg, 4.2 mmol) were dissolved in ethanol (15 mL), then concentrated hydrochloric acid (0.35 mL) was added, and the mixture was heated to reflux for 3 hours. The reaction mixture was cooled and filtered to obtain **WHE-109** (0.75 g, yield of 84%) as a white solid. ¹H NMR (600 MHz, CDCl₃) δ 10.15 (s, 2H), 7.24 - 7.19 (m, 1H), 7.01 (dd, *J =* 8.0, 7.1 Hz, 1H), 6.95-6.90 (m, 1H), 4.47 (d, *J =* 4.6 Hz, 2H), 3.99 (t, *J =* 5.7 Hz, 2H), 3.61 - 3.57 (m, 2H), 3.18 (t, *J* = 6.0 Hz, 2H), 2.98 (t, *J =* 6.1 Hz, 2H), 2.25 - 2.213 (m, 2H). HR-MS (ESI) *m*/*z* calculated for C₁₄H₁₇N₂⁺ [M+H]⁺: 213.1386, found: 213.1386.

Step 4: To a round-bottom flask were added **WHE-109** (1.35 g, 6.42 mmol) and TFA (13 mL), and the mixture was cooled in an ice-water bath. NaBH₃CN (1.2 g, 19.3 mmol) was then slowly added in batches. The reaction was removed from the ice-water bath and slowly warmed to room temperature, and stirred for 2 hours. After the reaction was completed, ice was added to quench the reaction, and the mixture was adjusted to an alkaline condition (pH = 14) using NaOH solution. Added water and the mixture was extracted three times with dichloromethane, and the organic phases were combined. The residue was concentrated to obtain the crude product **WHE-117** (1.3 g), which was directly used in the next step. ¹H NMR (800 MHz, CDCl₃) δ 6.92 (t, *J =* 6.7 Hz, 2H), 6.68 (t, *J =* 7.4 Hz, 1H), 3.44 - 3.35 (m, 2H), 3.34 - 3.26 (m, 3H), 3.16 (td, *J =* 13.0, 3.3 Hz, 1H), 2.76 - 2.67 (m, 3H), 2.54 - 2.51 (m, 1H), 2.28 - 2.23 (m, 1H), 2.19 - 2.16 (m, 1H), 2.15 - 2.09 (m, 2H). HR-MS (ESI) *m*/*z* calculated for C₁₄H₁₉N₂⁺ [M+H]⁺: 215.1543, found: 215.1546.

Step 5: The crude product **WHE-117** (1.3 g, 6.42 mmol) was dissolved in 1,4-dioxane (15 mL), then a solution of NaOH (500 mg, 12.8 mmol) in water (10 mL) was added, followed by addition of Boc₂O (1.54 g, 7.06 mmol). The reaction was stirred at room temperature for 2 hours. After the reaction was completed, water (15 mL) was added. The organic phase was isolated, and the aqueous phase was extracted with ethyl acetate. The organic phases were combined and concentrated. The residue was purified by flash silica gel chromatography to obtain **WHE-125** (1.8 g, two-step yield of 89%). ¹H NMR (800 MHz, CDCl₃) δ 6.96 (d, *J =* 7.2 Hz, 1H), 6.89 (d, *J =* 7.6 Hz, 1H), 6.66 (t, *J =* 7.5 Hz, 1H), 4.13-4.01 & 3.91 - 3.79 (m, 1H), 3.73 (d, *J =* 13.2 Hz, 1H), 3.49 - 3.45 (m, 1H), 3.31 - 3.27 (m, 1H), 3.26 - 3.09 (m, 2H), 3.09 - 2.97 & 2.85 - 2.80 (m, 1H), 2.73 (t, *J =* 6.5 Hz, 2H), 2.69-2.55 (m, 1H), 2.16 - 2.09 (m, 2H), 1.93 - 1.86 (m, 2H), 1.50 (s, 9H). HR-MS (ESI) *m*/*z* calculated for C₁₉H₂₇N₂O₂⁺ [M+H]⁺: 315.2067, found: 315.2067.

Step 6: Compound **WHE-125** was subjected to chiral resolution to obtain **WHE-125-p1** and **WHE-125-p2.**

Chiral analysis conditions: chiral column: OD-H (Daicel); column volume: 4.6 × 100 mm (5 µm packing); mobile phase: CO₂/isopropanol [0.5% ammonia methanol solution (7 M)] = 90/10; flow rate: 3.0 mL/min; wavelength: 214 nm; temperature: 40°C; HPLC instrument: UPCC (Waters); peak 1 (early peak) t_{R} = 1.672 min; peak 2 (later peak) t_{R} = 1.941 min.

Chiral preparation conditions: chiral column: OD (Daicel); column volume: 20 × 250 mm (10 µm packing); mobile phase: CO₂/isopropanol [0.5% ammonia methanol solution (7 M)] = 85/15; flow rate: 80 mL/min; wavelength: 214 nm; temperature: 35°C; HPLC instrument: SFC-150 (Waters).

**WHE-125-p1:** ¹H NMR (800 MHz, CDCl₃) δ 6.94 (d, *J =* 7.2 Hz, 1H), 6.87 (d, *J =* 7.5 Hz, 1H), 6.64 (t, *J =* 7.4 Hz, 1H), 4.10 - 4.05 & 3.89 - 3.81 (m, 1H), 3.74 - 3.68 (m, 1H), 3.46 - 3.35 (m, 1H), 3.29 - 3.23 (m, 1H), 3.23 - 3.11 (m, 2H), 3.06 - 2.96 & 2.81 - 2.74 (m, 1H), 2.72 - 2.68 (m, 2H), 2.65 - 2.55 (m, 1H), 2.16 - 2.09 (m, 2H), 1.94 - 1.91 (m, 1H), 1.89 - 1.83 (m, 1H), 1.50 (s, 9H). HR-MS (ESI) *m*/*z* calculated for C₁₉H₂₇N₂O₂⁺ [M+H]⁺: 315.2067; found: 315.2082. Specific rotation: [α]_{D}²⁵ = +128° (c = 0.1, CHCl₃).

**WHE-125-p2:** ¹H NMR (800 MHz, CDCl₃) δ 6.93 (d, *J =* 7.2 Hz, 1H), 6.87 (d, *J =* 7.6 Hz, 1H), 6.64 (t, *J =* 7.4 Hz, 1H), 4.10 - 3.99 & 3.87 - 3.79 (m, 1H), 3.72 (dt, *J* = 13.2, 4.4 Hz, 1H), 3.44 - 3.36 (m, 1H), 3.28 - 3.25 (m, 1H), 3.21 - 3.10 (m, 2H), 3.05 - 2.96 & 2.82-2.75 (m, 1H), 2.71 (dd, *J* = 8.2, 5.1 Hz, 2H), 2.64 - 2.56 (m, 1H), 2.14 - 2.07 (m, 2H), 1.93-1.88 (m, 1H), 1.87 - 1.79 (m, 1H), 1.49 (s, 9H). HR-MS (ESI) *m*/*z* calculated for C₁₉H₂₇N₂O₂⁺ [M+H]⁺: 315.2067, found: 315.2063. Specific rotation: [α]_{D}²⁵ = -115° (c = 0.1, CHCl₃).

The stereo-configurations of compounds **WHE-125-p1** and **WHE-125-p2** were determined by comparing their specific rotation values with those of starting materials **A** and WHF-108 with known stereo-configurations.

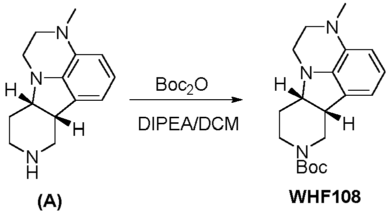

To a round-bottom flask was added **A** (50 mg, 0.13 mmol), which was dissolved in DCM (3 mL). Et₃N (1 mL) was added , followed by addition of Boc₂O (57 mg, 0.26 mmol). The reaction mixture was stirred at room temperature for 2 hours. After the reaction was completed, the solvent was removed under reduced pressure. The residue was purified by flash silica gel column chromatography (eluent: 0 to 30% ethyl acetate/petroleum ether) to obtain **WHF108** (32 mg, yield of 74%) as a colorless oil. ¹H NMR (800 MHz, CDCl₃) δ 6.72 - 6.61 (m, 1H), 6.58 - 6.48 (m, 1H), 6.46 - 6.34 (m, 1H), 4.16 - 4.07 & 3.97 - 3.86 (m, 1H), 3.85 - 3.76 (m, 1H), 3.65 - 3.53 (m, 1H), 3.42 - 3.23 (m, 3H), 3.20 - 3.05 (m, 2H), 2.80 - 2.60 (m, 5H), 1.80 - 1.71 (m, 2H), 1.49 (s, 9H). HR-MS (ESI) *m*/*z* calculated for C₁₉H₂₇N₃O₂⁺ [M+H]⁺: 329.2098; found: 329.2098. Specific rotation: [α]_{D}²⁵ = -53° (c = 0.1, CHCl₃); specific rotation of starting material **A:** [α]_{D}²⁵ = -165° (c = 0.1, CHCl₃).

Step 7: To a round-bottom flask was added **WHE-125-p2** (120 mg, 0.382 mmol), followed by the addition of the solvent dichloromethane (2 mL) and TFA (1 mL), and the mixture was stirred at room temperature for 1 hour. The mixture was concentrated under reduced pressure to remove the solvent. The residue was dissolved in DMSO (3 mL), added with 1-(2-bromoethyl)-2-methoxybenzene (100 mg, 0.465 mmol), and then added with *N,N-*diisopropylethylamine (3 mL). The reaction system was heated to 60°C and stirred overnight (18 hours). After the reaction was completed, water (50 mL) was added and the mixture was extracted three times ethyl acetate. The organic phases were combined and concentrated, and the residue was purified by flash silica gel column chromatography (eluent: 0 to 10% ethyl acetate/petroleum ether) to obtain **I-16** (70 mg, yield of 53%) as a brownish-yellow oil. ¹H NMR (800 MHz, CDCl₃) δ 7.22 - 7.20 (m, 1H), 7.16 (d, *J =* 7.4 Hz, 1H), 6.97 (d, *J =* 7.2 Hz, 1H), 6.91 - 6.89 (m, 2H), 6.86 (d, *J =* 8.2 Hz, 1H), 6.68 (t, *J =* 7.4 Hz, 1H), 3.82 (s, 3H), 3.33 - 3.29 (m, 2H), 3.27 - 3.24 (m, 1H), 3.03 - 3.01 (m, 1H), 2.91 - 2.86 (m, 2H), 2.84 - 2.80 (m, 1H), 2.78 - 2.71 (m, 2H), 2.65 - 2.53 (m, 3H), 2.42 (td, *J =* 11.2, 4.3 Hz, 1H), 2.19 - 2.07 (m, 3H), 2.07 - 1.99 (m, 2H). ¹³C NMR (201 MHz, CDCl₃) δ 157.62, 149.80, 131.09, 130.41, 128.68, 127.47, 126.84, 121.07, 120.58, 120.56, 118.68, 110.38, 64.13, 58.90, 55.91, 55.34, 48.88, 44.67, 40.88, 27.91, 24.91, 24.26, 23.16. HR-MS (ESI) *m*/*z* calculated for C₂₃H₂₉N₂O⁺ [M+H]⁺: 349.2274, found: 349.2276. Specific rotation: [α]_{D}²⁵ = -96° (c = 0.1, CHCl₃).

### Example 17: Preparation of (7aR,11aS)-10-(2-methoxyphenethyl)-5,6,7a,8,9,10,11,11a-octahydro-4H-pyrido[3',4':4,5]pyrrolo[3,2,1-ij]quinoline (compound I-17)

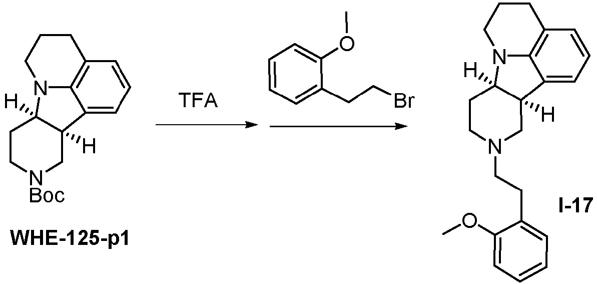

Following the method described in step 7 of Example 16, compound **WHE-125-p1** was subjected to Boc deprotection and then alkylated with 1-(2-bromoethyl)-2-methoxybenzene to obtain compound **I-17** (90 mg, yield of 82%) as a brownish-yellow oil. ¹H NMR (800 MHz, CDCl₃) δ 7.22 - 7.17 (m, 1H), 7.14 (d, *J =* 7.6 Hz, 1H), 6.95 (d, *J =* 7.2 Hz, 1H), 6.88 (t, *J =* 7.5 Hz, 2H), 6.84 (d, *J =* 8.1 Hz, 1H), 6.66 (t, *J =* 7.4 Hz, 1H), 3.81 (s, 3H), 3.32 - 3.26 (m, 2H), 3.23 - 3.20 (m, 1H), 3.00 - 2.96 (m, 1H), 2.88 - 2.82 (m, 2H), 2.81 - 2.76 (m, 1H), 2.73 - 2.71 (m, 2H), 2.61 - 2.51 (m, 3H), 2.37 (td, *J =* 11.7, 3.3 Hz, 1H), 2.18 - 1.95 (m, 5H). ¹³C NMR (201 MHz, CDCl₃) δ 157.62, 149.80, 131.17, 130.38, 128.79, 127.43, 126.80, 121.04, 120.54, 120.53, 118.63, 110.38, 64.16, 58.96, 56.00, 55.33, 48.91, 44.65, 40.94, 27.99, 24.98, 24.26, 23.15. HR-MS (ESI) *m*/*z* calculated for C₂₃H₂₉N₂O⁺ [M+H]⁺: 349.2274, found: 349.2282. Specific rotation: [α]_{D}²⁵ = +104° (c = 0.1, CHCl₃).

### Example 18: Preparation of 10-(3-(2-methoxyphenyl)propyl)-5,6,7a,8,9,10,11,11a-octahydro-4H-pyrido[3',4':4,5]pyrrolo[3,2,1-ij]quinoline (compound I-18)

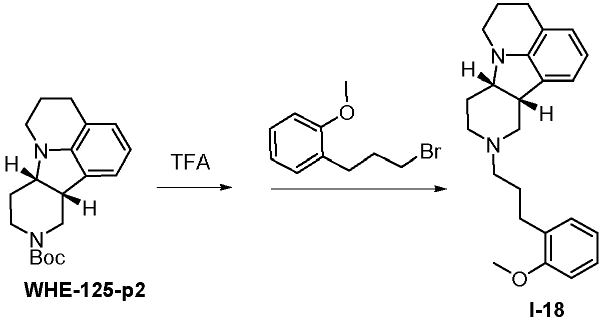

Following the method described in step 7 of Example 16, compound **WHE-125-p2** was subjected to Boc deprotection and then alkylated with 1-(3-bromopropyl)-2-methoxybenzene to obtain compound **I-18** (50 mg, yield of 50%) as a brownish-yellow oil. ¹H NMR (800 MHz, CDCl₃) δ 7.20 (t, *J =* 7.8 Hz, 1H), 7.16 (d, *J =* 7.4 Hz, 1H), 6.94 (d, *J =* 7.2 Hz, 1H), 6.92 - 6.88 (m, 2H), 6.86 (d, *J =* 8.2 Hz, 1H), 6.66 (t, *J =* 7.4 Hz, 1H), 3.84 (s, 3H), 3.31- 3.28 (m, 2H), 3.21 - 3.18 (m, 1H), 2.90 - 2.88 (m, 1H), 2.75 - 2.69 (m, 3H), 2.67 - 2.62 (m, 2H), 2.56 (td, *J =* 10.4, 3.1 Hz, 1H), 2.46 - 2.37 (m, 2H), 2.29 (td, *J =* 11.7, 3.2 Hz, 1H), 2.18 - 2.08 (m, 2H), 2.03 - 1.92 (m, 3H), 1.87 - 1.83 (m, 2H). ¹³C NMR (201 MHz, CDCl₃) δ 157.51, 149.75, 131.19, 130.68, 129.86, 127.07, 126.75, 120.95, 120.48, 120.41, 118.58, 110.25, 64.17, 58.69, 56.14, 55.30, 49.04, 44.59, 40.99, 28.35, 27.10, 25.03, 24.26, 23.15. HR-MS (ESI) *m*/*z* calculated for C₂₄H₃₁N₂O⁺ [M+H]⁺: 363.2431, found: 363.2439. Specific rotation: [α]_{D}²⁵ = -108° (c = 0.1, CHCl₃).

### Example 19: Preparation of (7aR,11aS)-10-(3-(2-methoxyphenyl)propyl)-5,6,7a,8,9,10,11,11a-octahydro-4H-pyrido[3',4':4,5]pyrrolo[3,2,1-ij]quinoline (compound I-19)

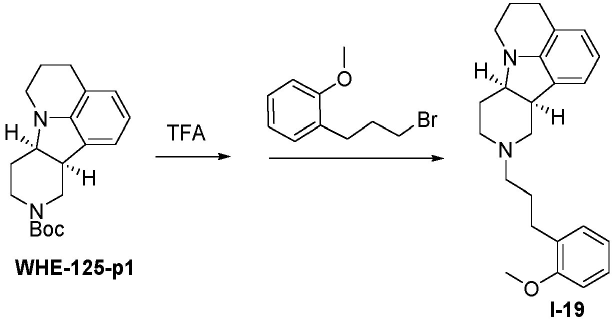

Following the method described in step 7 of Example 16, compound **WHE-125-p1** was subjected to Boc deprotection and then alkylated with 1-(3-bromopropyl)-2-methoxybenzene to obtain compound **I-19** (48 mg, yield of 46%) as a brownish-yellow oil. ¹H NMR (800 MHz, CDCl₃) δ 7.09 (t, *J =* 7.7 Hz, 1H), 7.05 (d, *J =* 7.4 Hz, 1H), 6.83 (d, *J =* 7.2 Hz, 1H), 6.80 - 6.77 (m, 2H), 6.75 (d, *J =* 8.2 Hz, 1H), 6.55 (t, *J =* 7.4 Hz, 1H), 3.73 (s, 3H), 3.20 - 3.16 (m, 2H), 3.12 - 3.09 (m, 1H), 2.82 - 2.78 (m, 1H), 2.67 - 2.58 (m, 3H), 2.55 - 2.53 (m, 2H), 2.45 (td, *J* = 10.3, 3.3 Hz, 1H), 2.36 - 2.29 (m, 2H), 2.22 - 2.18 (m, 1H), 2.06 - 1.98 (m, 2H), 1.95 - 1.84 (m, 3H), 1.77 - 1.73 (m, 2H). HR-MS (ESI) *m*/*z* calculated for C₂₄H₃₁N₂O⁺ [M+H]⁺: 363.2431, found: 363.2438, specific rotation: [α]_{D}²⁵ = +101° (c = 0.1, CHCl₃).

### Example 20: Preparation of (6bR,10aS)-8-(2-methoxyphenethyl)-1,2,6b,7,8,9,10,10a-octahydro-[1,4]oxazino[2,3,4-hi]pyrido[4,3-b]indole (compound I-20)

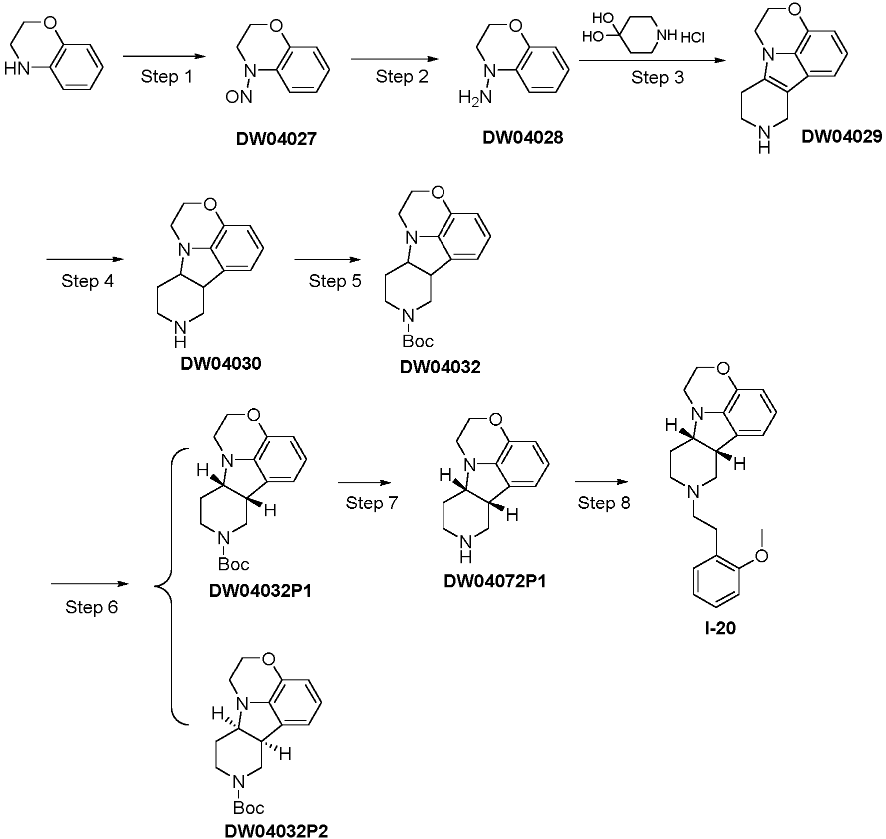

Step 1: To a round-bottom flask was added benzomorpholine (2.6 g, 20 mmol), which was dissolved in tetrahydrofuran (40 mL), and the reaction system was stirred at 0°C. 3 M hydrochloric acid (13 mL) was slowly added dropwise, and the reaction mixture continued to be stirred at 0°C. After 1 hour, a solution of sodium nitrite (3.45 g, 50 mmol) in water (10 mL) was added dropwise. The reaction mixture continued to be stirred at room temperature overnight. Upon the completion of the reaction (TLC), the reaction mixture was distilled under reduced pressure to remove most of the solvent, and extracted three times with ethyl acetate. The organic phases were combined, washed with saturated brine, and dried over anhydrous sodium sulfate. The organic phase was concentrated to obtain a crude product, which was purified by flash silica gel column chromatography (eluent: 0 to 25% ethyl acetate/petroleum ether) to obtain **DW04027** (3.0 g, yield of 91%) as a yellow solid. ¹H NMR (800 MHz, CDCl₃) δ 8.10 (dd, *J =* 8.2, 1.4 Hz, 1H), 7.24 - 7.18 (m, 1H), 7.09 - 7.05 (m, 1H), 7.03 (dd, *J =* 8.2, 1.2 Hz, 1H), 4.28 - 4.22 (m, 2H), 4.08 - 4.04 (m, 2H). HRMS (ESI) *m*/*z* calculated for C₈H₉N₂O₂⁺ [M+H]⁺: 165.0659, found: 165.0672.

Step 2: To a round-bottom flask was added **DW04027** (1.6 g, 10 mmol), which was dissolved in a mixture of acetic acid and water (1:1, 20 mL), and the reaction was stirred at 0 to 5°C. Zinc powder (2.6 g, 40 mmol) was slowly added in batches. The mixture was stirred at room temperature for 1 hour. The zinc powder was filtered off. The filtrate was adjusted to pH 9.0 with 2 N NaOH, and extracted three times with ethyl acetate. The organic phases were combined, washed with saturated brine, and dried over anhydrous sodium sulfate. The organic phase was concentrated to obtain a crude product, which was purified by flash silica gel column chromatography (eluent: 0 to 35% ethyl acetate/petroleum ether) to obtain **DW04028** (1.2 g, yield of 80%) as a pale yellow solid. ¹H NMR (800 MHz, CDCl₃) δ 7.11 (dd, *J =* 8.0, 1.0 Hz, 1H), 6.89 - 6.87 (m, 1H), 6.78 - 6.73 (m, 2H), 4.40 - 4.32 (m, 2H), 4.12 (brs, 2H), 3.43 -3.38 (m, 2H). HRMS (ESI) *m*/*z* calculated for C₈H₁₁N₂O⁺ [M+H]⁺: 151.0866, found: 151.0871.

Step 3: To a round-bottom flask were added **DW04028** (1.5 g, 10 mmol) and 4,4-piperidinediol hydrochloride (1.2 g, 10 mmol). The mixture was dissolved in ethanol (20 mL). Concentrated hydrochloric acid (1 mL) was slowly added dropwise. After the dropwise addition was completed, the reaction system was heated to 90°C and refluxed with stirring for 3 hours. The reaction mixture was cooled to room temperature and filtered to obtain **DW04029** (1.6 g, yield of 73%) as an off-white solid. HRMS(ESI) *m*/*z* calculated for C₁₃H₁₅N₂O⁺ [M+H]⁺: 215.1179; found: 215.1185.

Step 4: To a round-bottom flask was added **DW04029** (2.1 g, 10 mmol), which was dissolved in trifluoroacetic acid (20 mL), and the reaction was stirred at 0°C. Sodium cyanoborohydride (1.89 g, 30 mmol) was added in batches, and the mixture was stirred at room temperature for 2 hours. After the reaction was completed, the mixture was distilled under reduced pressure to remove most of the trifluoroacetic acid, adjusted with 2 N sodium hydroxide aqueous solution to pH 9.0, and extracted three times with ethyl acetate. The organic phases were combined, washed with saturated brine, and dried over anhydrous sodium sulfate. The organic phase was concentrated to obtain a crude product, which was purified by flash silica gel column chromatography (eluent: 0 to 10% methanol/dichloromethane) to obtain **DW04030** (1.2 g, yield of 61%) as a yellow oil. ¹H NMR (800 MHz, CDCl₃) δ 6.78-6.73 (m, 2H), 6.73 - 6.71 (m, 1H), 4.51 (d, *J* = 11.1 Hz, 1H), 4.48 - 4.43 (m, 1H), 3.56 - 3.54 (m, 1H), 3.48 - 3.44 (m, 2H), 3.42 - 3.40 (m, 1H), 3.35 - 3.33 (m, 2H), 2.93 (q, *J =* 11.8 Hz, 1H), 2.82 (td, *J =* 10.5, 2.7 Hz, 1H), 2.30 - 2.21 (m, 2H). HRMS (ESI) *m*/*z* calculated for C₁₃H₁₇N₂O⁺ [M+H]⁺: 217.1335, found: 217.1351.

Step 5: To a round-bottom flask were added **DW04030** (2.1 g, 10 mmol) and di-*tert-*butyl dicarbonate (2.2 g, 10 mmol). The mixture was dissolved in dioxane (25 mL) at room temperature with stirring. 1 N sodium hydroxide aqueous solution (10 mL) was then added , and the reaction was stirred at room temperature overnight. After the reaction was completed, the mixture was extracted three times with ethyl acetate. The organic phases were combined, washed with saturated brine, and dried over anhydrous sodium sulfate. The organic phase was concentrated to obtain the crude product **DW04032** (3.0 g, yield of 94%) as an oil. HRMS (ESI) *m*/*z* calculated for C₁₈H₂₅N₂O₃⁺ [M+H]⁺: 317.1860, found: 317.1871.

Step 6: **DW04032** was subjected to chiral resolution.

Chiral analysis method: instrument: Shimadzu; chiral column: OJ-H (Daicel); column volume: 4.6 × 250 mm (5 µm packing); temperature: 40°C; mobile phase: n-hexane (0.1% diethylamine): ethanol (0.1% diethylamine) = 70:30; flow rate: 1.0 mL/min; detection wavelength: 214 nm and 254 nm.

Chiral preparation method: instrument: Gilson-281; chiral column: OJ (Daicel); column volume: 20 × 250 mm (10 µm packing); temperature: 35°C; mobile phase: n-hexane (0.1% diethylamine): ethanol (0.1% diethylamine) = 75:25; flow rate: 40.0 mL/min; detection wavelength: 214 nm. **DW04032P1** (e.e. 100%) and **DW04032P2** (e.e. 100%) were prepared.

Step 7: To a round-bottom flask was added **DW04032P1** (310 mg, 1 mmol), which was dissolved in dichloromethane (5 mL) with stirring, and then trifluoroacetic acid (1 mL) was added. The reaction was stirred at room temperature for 2 hours. After the reaction was completed, the reaction system was adjusted with 2 N sodium hydroxide aqueous solution to pH 9.0, and extracted three times with ethyl acetate. The organic phases were combined and washed with saturated brine. The organic phase was concentrated to obtain the crude product **DW04072P1** (208 mg, yield of 96%) as a pale yellow solid. HRMS (ESI) *m*/*z* calculated for C₁₃H₁₇N₂O⁺ [M+H]⁺: 217.1335, found: 217.1343.

Step 8: Following the method described in step 7 of Example 16, **DW04072P1** was alkylated with 1-(2-bromoethyl)-2-methoxybenzene to obtain **I-20** (80 mg, yield of 49%) as a pale yellow oil. ¹H NMR (800 MHz, CDCl₃) δ 7.10 (td, *J =* 8.0, 1.6 Hz, 1H), 7.06 (dd, *J =* 7.4, 1.4 Hz, 1H), 6.79 (t, *J =* 7.4 Hz, 1H), 6.75 (d, *J =* 8.1 Hz, 1H), 6.65 (d, *J =* 7.0 Hz, 1H), 6.58 - 6.54 (m, 2H), 4.39 - 4.34 (m, 2H), 3.72 (s, 3H), 3.21 (dt, *J =* 10.7, 2.0 Hz, 1H), 3.20-3.18 (m, 1H), 3.17 - 3.13 (m, 1H), 2.96 - 2.93 (m, 1H), 2.79 - 2.75 (m, 2H), 2.75 - 2.71 (m, 1H), 2.69 - 2.64 (m, 1H), 2.54 - 2.46 (m, 2H), 2.31 (td, *J =* 11.5, 3.7 Hz, 1H), 2.05 (t, *J* = 11.3 Hz, 1H), 1.95 -1.86 (m, 2H). ¹³C NMR (201 MHz, CDCl₃) δ 157.60, 143.52, 138.01, 132.83, 130.35, 128.73, 127.42, 120.52, 120.36, 115.86, 113.21, 110.36, 66.85, 65.65, 60.48, 58.95, 55.91, 55.31, 48.93, 44.73, 41.58, 28.02. HRMS (ESI) *m*/*z* calculated for C₂₂H₂₇N₂O₂⁺ [M+H]⁺: 351.2067, found: 351.2079. Specific rotation: [α]_{D}²⁵ = -11° (c = 0.1, CHCl₃), the optical rotation direction is consistent with that of compound **I-3,** being levorotatory, indicating an identical chiral configuration.

### Example 21: Preparation of (6bS,10aR)-8-(2-methoxyphenethyl)-1,2,6b,7,8,9,10,10a-octahydro-[1,4]oxazino[2,3,4-hi]pyrido[4,3-b]indole (compound I-21)

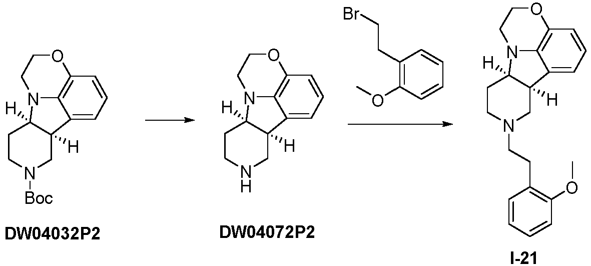

Following the method described in step 7 of Example 20, **DW04032P2** was used as the starting material to prepare **DW04072P2** (180 mg, yield of 93%) as a pale yellow solid. HRMS (ESI) *m*/*z* calculated for C₁₃H₁₇N₂O⁺ [M+H]⁺: 217.1335, found: 217.1348.

Following the method described in step 8 of Example 20, **DW04072P2** was alkylated with 1-(2-bromoethyl)-2-methoxybenzene to obtain **I-21** (83 mg, yield of 51%) as a pale yellow oil. ¹H NMR (800 MHz, CDCl₃) δ 7.10 (td, *J =* 8.0, 1.6 Hz, 1H), 7.06 (td, *J =* 7.1, 2.0 Hz, 1H), 6.82 - 6.78 (m, 1H), 6.76 (d, *J =* 8.1 Hz, 1H), 6.65 (d, *J =* 7.0 Hz, 1H), 6.59 - 6.54 (m, 2H), 4.41 - 4.34 (m, 2H), 3.72 (s, 3H), 3.22 (dt, *J =* 10.7, 2.1 Hz, 1H), 3.20 - 3.18 (m, 1H), 3.18 - 3.14 (m, 1H), 2.98 - 2.94 (m, 1H), 2.78 (dt, *J =* 11.2, 6.0 Hz, 2H), 2.76 - 2.73 (m, 1H), 2.69 - 2.65 (m, 1H), 2.55 - 2.47 (m, 2H), 2.32 (td, *J =* 11.6, 3.3 Hz, 1H), 2.06 (t, *J =* 11.2 Hz, 1H), 1.95 - 1.87 (m, 2H). ¹³C NMR (201 MHz, CDCl₃) δ 157.61, 143.53, 138.01, 132.80, 130.36, 128.69, 127.45, 120.53, 120.39, 115.88, 113.23, 110.37, 66.86, 65.64, 60.49, 58.92, 55.87, 55.32, 48.92, 44.75, 41.55, 28.01. HRMS (ESI) *m*/*z* calculated for C₂₂H₂₇N₂O₂⁺ [M+H]⁺: 351.2067, found: 351.2082. Specific rotation: [α]_{D}²⁵ = +41° (c = 0.1, CHCl₃), the optical rotation direction is opposite to that of compound **I-3,** being dextrorotatory, indicating an opposite chiral configuration.

### Example 22: Preparation of (6bR,10aS)-8-(3-(2-methoxyphenyl)propyl)-1,2,6b,7,8,9,10,10a-octahydro-[1,4]oxazino[2,3,4-hi]pyrido[4,3-b]indole (compound I-22)

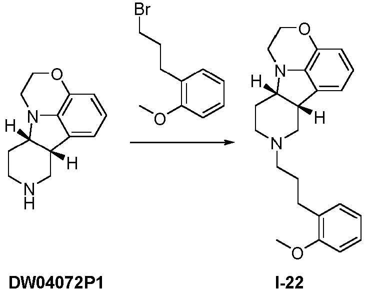

Following the method described in step 7 of Example 16, **DW04072P1** was alkylated with 1-(3-bromopropyl)-2-methoxybenzene to obtain **I-22** (105 mg, yield of 58%) as a pale yellow oil. ¹H NMR (800 MHz, CDCl₃) δ 7.17 (td, *J* = 8.0, 1.6 Hz, 1H), 7.14 - 7.12 (m, 1H), 6.87 (t, *J =* 7.3 Hz, 1H), 6.83 (d, *J =* 8.1 Hz, 1H), 6.69 (t, *J =* 6.7 Hz, 1H), 6.66 - 6.60 (m, 2H), 4.47 - 4.40 (m, 2H), 3.81 (s, 3H), 3.28 (dt, *J =* 10.7, 2.1 Hz, 1H), 3.27 - 3.24 (m, 1H), 3.24-3.20 (m, 1H), 2.96 - 2.94 (m, 1H), 2.76 - 2.73 (m, 2H), 2.64 - 2.60 (m, 2H), 2.48 - 2.39 (m, 2H), 2.31 (td, *J =* 11.6, 2.4 Hz, 1H), 2.07 - 2.02 (m, 2H), 2.01 - 1.96 (m, 1H), 1.95 - 1.94 (m, 1H), 1.86 - 1.81 (m, 2H). ¹³C NMR (201 MHz, CDCl₃) δ 157.52, 143.55, 137.96, 132.73, 130.54, 129.88, 127.15, 120.44, 120.42, 115.85, 113.26, 110.28, 66.86, 65.59, 60.34, 58.52, 55.82, 55.32, 48.90, 44.72, 41.43, 28.31, 26.89. HRMS (ESI) *m*/*z* calculated for C₂₃H₂₉N₂O₂⁺ [M+H]⁺: 365.2224, found: 365.2239. Specific rotation: [α]_{D}²⁵ = -10° (c = 0.1, CHCl₃), the optical rotation direction is consistent with that of compound **I-10,** being levorotatory, indicating an identical chiral configuration.

### Example 23: Preparation of (6bS,10aR)-8-(3-(2-methoxyphenyl)propyl)-1,2,6b,7,8,9,10,10a-octahydro-[1,4]oxazino[2,3,4-hi]pyrido[4,3-b]indole (compound I-23)

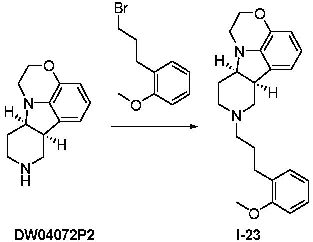

Following the method described in step 7 of Example 16, **DW04072P2** was alkylated with 1-(3-bromopropyl)-2-methoxybenzene to obtain **I-23** (83 mg, yield of 51%) as a pale yellow oil. ¹H NMR (800 MHz, CDCl₃) δ 7.17 (td, *J =* 8.1, 1.5 Hz, 1H), 7.13 - 7.11 (m, 1H), 6.87 (t, *J =* 7.3 Hz, 1H), 6.83 (d, *J =* 8.2 Hz, 1H), 6.70 (d, *J =* 7.0 Hz, 1H), 6.66 - 6.60 (m, 2H), 4.43 (dd, *J =* 11.3, 6.1 Hz, 2H), 3.80 (d, *J* = 18.3 Hz, 3H), 3.28 (dt, *J =* 10.7, 2.0 Hz, 1H), 3.26 - 3.22 (m, 2H), 2.99 - 2.96 (m, 1H), 2.80 - 2.72 (m, 2H), 2.64 - 2.60 (m, 2H), 2.50 - 2.40 (m, 2H), 2.34 (t, *J =* 10.3 Hz, 1H), 2.07 (t, *J =* 11.2 Hz, 1H), 2.04 - 2.00 (m, 1H), 1.97 - 1.91 (m, 1H), 1.88 - 1.81 (m, 2H). ¹³C NMR (201 MHz, CDCl₃) δ 157.53, 143.58, 137.94, 130.42, 129.90, 127.20, 124.11, 120.49, 120.47, 115.89, 113.33, 110.31, 66.87, 65.53, 58.44, 55.34, 48.83, 44.74, 41.28, 31.57, 30.32, 29.85, 28.29. HRMS (ESI) *m*/*z* calculated for C₂₃H₂₉N₂O₂⁺ [M+H]⁺: 365.2224, found: 365.2225. Specific rotation: [α]_{D}²⁵ = +49° (c = 0.1, CHCl₃), the optical rotation direction is opposite to that of compound **I-10,** being dextrorotatory, indicating an opposite chiral configuration.

### Example 24: Preparation of (8aS,12aR)-11-(2-methoxyphenethyl)-6,7,8a,9,10,11,12,12a-octahydro-5H-pyrido[4,3-b][1,4]thiazepino[2,3,4-hi]indole (compound I-24)

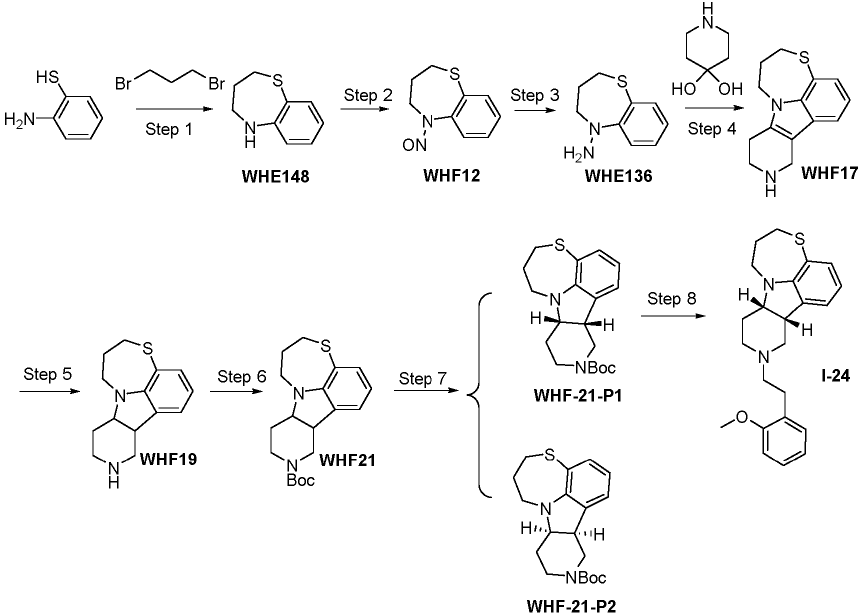

Step 1: To a round-bottom flask were added 2-aminobenzenethiol (2.5 g, 20 mmol), 1,3-dibromopropane (6.03 g, 30 mmol), and K₂CO₃ (8.28 g, 60 mmol). Under a nitrogen atmosphere, DMF (20 mL) was added. The reaction system was heated to 65°C and stirred overnight. After the reaction was completed, the reaction mixture was concentrated under reduced pressure to remove DMF. The residue was purified by flash silica gel column chromatography (eluent: 0 to 20% ethyl acetate/petroleum ether) to obtain **WHE148** (1.41 g, yield of 50%) as a brownish-red oil. ¹H NMR (800 MHz, CDCl₃) δ 7.40 (dd, *J =* 7.6, 1.5 Hz, 1H), 7.08 - 7.06 (m, 1H), 6.82 - 6.80 (m, 1H), 6.77 (d, *J =* 7.8 Hz, 1H), 3.30 - 3.27 (m, 2H), 2.86 - 2.83 (m, 2H), 2.12 - 2.07 (m, 2H). HR-MS (ESI) *m*/*z* calculated for C₉H₁₂NS⁺ [M+H]⁺: 166.0685, found: 166.0688.

Step 2: The starting material **WHE-148** (1.59 g, 9.63 mmol) was dissolved in HOAc/H₂O (15 mL/5 mL) in a round-bottom flask. The mixture was cooled in an ice-water bath, and NaNO₂ (797 mg, 11.6 mmol) was added. The reaction was slowly warmed to room temperature and stirred overnight. After the reaction was completed, the solvent was removed under reduced pressure. The residue was dissolved in ethyl acetate and washed once with saturated NaHCO₃ aqueous solution. The aqueous phase was extracted with ethyl acetate. The organic phases were combined, concentrated, and purified by flash silica gel column chromatography to obtain **WHF-12** (1.53 g, 82%) as a brownish-yellow oil. ¹H NMR (800 MHz, CDCl₃) δ 7.55 (dd, *J =* 7.8, 1.5 Hz, 1H), 7.47 (dd, *J =* 7.7, 1.6 Hz, 1H), 7.35 - 7.27 (m, 2H), 4.20 (t, *J* = 6.0 Hz, 2H), 2.91 - 2.86 (m, 2H), 2.16 (p, *J* = 6.0 Hz, 2H). HR-MS (ESI) *m*/*z* calculated for C₉H₁₁N₂OS⁺ [M+H]⁺: 195.0587, found: 195.0587.

Step 3: Under a nitrogen atmosphere, LiAlH₄ (600 mg, 15.8 mmol) was suspended in anhydrous THF (20 mL). The reaction mixture was cooled in an ice-water bath, and a solution of **WHE-148** (1.53 g, 7.89 mmol) in anhydrous THF (5 mL) was slowly added. The reaction mixture continued to be stirred in an ice-water bath for 30 minutes, and then heated to 50°C and stirred overnight. After the reaction was completed, the reaction mixture was cooled in an ice-water bath, potassium sodium tartrate aqueous solution was added to quench the reaction. After the filtration, water was added to the filtrate, and the organic phase was isolated. The aqueous phase was extracted three times with ethyl acetate, and the organic phases were combined. The organic phase was concentrated and purified by flash silica gel column chromatography to obtain **WHE-136** (1.1 g, yield of 77%) as an orange-yellow oil. HR-MS (ESI) *m*/*z* calculated for C₉H₁₃N₂S⁺ [M+H]⁺: 181.0794, found: 181.0794.

Step 4: **WHE-136** was dissolved in ethanol (10 mL), and 4,4-dihydroxypiperidine hydrochloride (1.85 g, 12.1 mmol) and hydrochloric acid (0.6 mL) were added. The reaction was heated to reflux overnight. After the reaction was completed, ethanol was removed under reduced pressure. Water was added and the mixture was extracted with ethyl acetate. The organic phases were combined, washed once with saturated NaHCO₃ aqueous solution, then concentrated under reduced pressure, and purified by flash silica gel column chromatography to obtain **WHF-19** (1.4 g, yield of 94%) as a pale yellow solid. ¹H NMR (800 MHz, MeOD) δ 7.24 (dd, *J =* 7.8, 1.0 Hz, 1H), 7.02 (dd, *J =* 7.4, 1.0 Hz, 1H), 6.90 (t, *J =* 7.6 Hz, 1H), 4.61-4.57 (m, 2H), 4.38 (d, *J =* 1.5 Hz, 2H), 3.63 (t, *J =* 6.2 Hz, 2H), 3.35 - 3.31 (m, 2H), 3.10 (td, *J* = 6.5, 3.3 Hz, 2H), 2.38 - 2.32 (m, 2H). HR-MS (ESI) *m*/*z* calculated for C₁₄H₁₇N₂S⁺ [M+H]⁺: 245.1107, found: 245.1108.

Step 5: Compound **WHF-17** (1.09 g, 6.05 mmol) was dissolved in trifluoroacetic acid, and the reaction mixture was cooled in an ice-water bath. NaBH₃CN (1.1 g, 17.2 mmol) was then added in batches within 30 minutes, and the reaction was stirred at room temperature for 2 hours. After the reaction was completed, ice was added, and the pH was adjusted to alkaline with NaOH aqueous solution. The reaction mixture was extracted three times with ethyl acetate. The organic phases were combined and concentrated to obtain the crude product **WHF-19** (1.5 g) as a brownish-yellow solid, which was directly used in the next step.

Step 6: The crude product **WHF-19** (1.5 g, 6.1 mmol) from the previous step was dissolved in dichloromethane (10 mL), then Et₃N (2 mL) Boc₂O (2.6 g, 12.2 mmol) were added. The reaction system was stirred at room temperature for 2 hours. After the reaction was completed, the solvent was removed under reduced pressure. The residue was purified by flash silica gel column chromatography to obtain compound **WHF-21** (1.4 g, two-step yield of 67%). ¹H NMR (800 MHz, CDCl₃) δ 6.94 (d, *J* = 7.2 Hz, 1H), 6.87 (d, *J* = 7.6 Hz, 1H), 6.64 (t, *J =* 7.5 Hz, 1H), 4.14 - 3.97 & 3.87 - 3.79 (m, 1H), 3.71 (d, *J =* 13.1 Hz, 1H), 3.45 - 3.35 (m, 1H), 3.28 - 3.25 (m, 1H), 3.23 - 3.06 (m, 2H), 3.06 - 2.95 & 2.83 - 2.75 (m, 1H), 2.71 (t, *J =* 6.5 Hz, 2H), 2.64 - 2.57 (m, 1H), 2.15 - 2.07 (m, 2H), 1.92 - 1.88 (m, 1H), 1.86 - 1.79 (m, 1H), 1.47 (s, 9H). HR-MS (ESI) *m*/*z* calculated for C₁₉H₂₇N₂O₂S⁺ [M+H]⁺: 347.1788, found: 347.1788.

**Step 7:** Compound **WHF-21** was subjected to chiral resolution.

Chiral analysis conditions: chiral column: IG (Daicel); column volume: 4.6 × 100 mm (5 µm packing); mobile phase: CO₂/MeOH [0.2% ammonia methanol solution (7 M)] = 85/15; flow rate: 3.0 mL/min; detection wavelength: 214 nm; temperature: 40°C; HPLC instrument: UPCC (Waters); peak 1 (early peak) t_{R} = 1.926 min; peak 2 (later peak) t_{R} = 2.344 min.

Chiral preparation conditions: chiral column: IG (Daicel); column volume: 20 × 250 mm (10 µm packing); mobile phase: CO₂/MeOH [0.2% ammonia methanol solution (7 M)] = 75/25; flow rate: 100 mL/min; detection wavelength: 214 nm; temperature: 35°C; HPLC instrument: SFC-150 (Waters).

**WHF-21-p1:** ¹H NMR (800 MHz, CDCl₃) δ 6.93 (d, *J* = 7.8 Hz, 1H), 6.86 (d, *J* = 7.2 Hz, 1H), 6.60 (t, *J =* 7.5 Hz, 1H), 3.93 - 3.86 (s, 0.5H), 3.79 - 3.75 (m, 1H), 3.73 - 3.63 (m, 0.5H), 3.62 - 3.53 (m, 1H), 3.53 - 3.38 (m, 2H), 3.38 - 3.24 (m, 1H), 3.22 - 3.17 (m, 1H), 3.13 - 3.05 (m, 1.5H), 2.94 - 2.88 (m, 1H), 2.87 - 2.78 (m, 0.5H), 2.11 - 2.06 (m, 1H), 2.03 - 1.99 (m, 1H), 1.85 - 1.83 (m, 2H), 1.42 (s, 9H). Specific rotation: [α]_{D}²⁵ = -143° (c = 0.1, CHCl₃).

**WHF-21-p2:** ¹H NMR (800 MHz, CDCl₃) δ 6.96 - 6.93 (m, 1H), 6.87 (d, *J =* 7.2 Hz, 1H), 6.61 (t, *J =* 7.5 Hz, 1H), 3.94 - 3.88 (m, 0.5H), 3.81 - 3.77 (m, 1H), 3.70 - 3.64 (m, 0.5H), 3.62 - 3.55 (m, 1H), 3.53 - 3.39 (m, 2H), 3.39 - 3.16 (m, 2H), 3.14 - 3.07 (m, 1H), 3.03 - 3.00 (m, 0.5H), 2.94 - 2.90 (m, 1H), 2.86 - 2.80 (m, 0.5H), 2.12 - 2.07 (m, 1H), 2.06 - 1.99 (m, 1H), 1.86 - 1.80 (m, 2H), 1.43 (s, 9H). Specific rotation: [α]_{D}²⁵ = +135° (c = 0.1, CHCl₃).

The stereo-configurations of compounds **WHF-21-p1** and **WHF-21-p2** were determined by comparing their specific rotation values with those of starting materials A and **WHF-108** with known stereo-configurations respectively.

Step 8: Following the method described in step 7 of Example 16, compound **WHF-21-p1** was subjected to Boc deprotection and then alkylated with 1-(2-bromoethyl)-2-methoxybenzene to obtain **I-24** (51 mg, yield of 59%) as an orange-yellow oil. ¹H NMR (800 MHz, CDCl₃) δ 7.19 (t, *J =* 7.7 Hz, 1H), 7.14 (d, *J =* 7.4 Hz, 1H), 6.96 (d, *J =* 7.8 Hz, 1H), 6.91 - 6.86 (m, 2H), 6.84 (d, *J* = 8.1 Hz, 1H), 6.64 (t, *J =* 7.5 Hz, 1H), 3.87 - 3.82 (m, 1H), 3.81 (s, 3H), 3.59 - 3.55 (m, 1H), 3.29 - 3.28 (m, 1H), 3.23 - 3.19 (m, 1H), 3.07 - 3.05 (m, 1H), 2.95 - 2.92 (m, 1H), 2.91 - 2.88 (m, 1H), 2.84 (t, *J =* 8.2 Hz, 2H), 2.80 - 2.77 (m, 1H), 2.59 - 2.51 (m, 2H), 2.39 - 2.33 (m, 1H), 2.15 - 2.11 (m, 1H), 2.06 - 1.98 (m, 1H), 1.98 - 1.93 (m, 3H). ¹³C NMR (201 MHz, CDCl₃) δ 157.59, 152.31, 133.86, 130.37, 128.84, 128.66, 127.45, 121.31, 120.53, 119.79, 119.72, 110.37, 63.98, 58.96, 56.52, 55.33, 49.10, 47.22, 40.94, 32.03, 30.61, 28.04, 25.73. HR-MS (ESI) *m*/*z* calculated for C₂₃H₂₉N₂OS⁺ [M+H]⁺: 381.1995, found: 381.2013. Specific rotation: [α]_{D}²⁵ = -134° (c = 0.1, CHCl₃).

### Example 25: Preparation of (8aR,12aS)-11-(2-methoxyphenethyl)-6,7,8a,9,10,11,12,12a-octahydro-5H-pyrido[4,3-b][1,4]thiazepino[2,3,4-hi]indole (compound I-25)

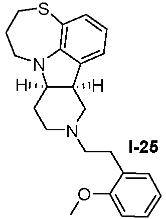

Following the method described in step 7 of Example 16, compound **WHF-21-p2** was subjected to Boc deprotection and then alkylated with 1-(2-bromoethyl)-2-methoxybenzene to obtain **I-25** (62 mg, yield of 67%) as an orange-yellow oil. ¹H NMR (800 MHz, CDCl₃) δ 7.20 - 7.18 (m, 1H), 7.14 (d, *J =* 7.4 Hz, 1H), 6.96 (d, *J =* 7.8 Hz, 1H), 6.91 - 6.86 (m, 2H), 6.84 (d, *J* = 8.1 Hz, 1H), 6.64 (t, *J* = 7.5 Hz, 1H), 3.86 - 3.82 (m, 1H), 3.81 (s, 3H), 3.60 - 3.56 (m, 1H), 3.30 - 3.28 (m, 1H), 3.23 - 3.20 (m, 1H), 3.08 - 3.05 (m, 1H), 2.97 - 2.88 (m, 2H), 2.84 (t, *J =* 8.2 Hz, 2H), 2.81 - 2.76 (m, 1H), 2.60 - 2.51 (m, 2H), 2.38 - 2.34 (m, 1H), 2.17-2.12 (m, 1H), 2.06 - 1.99 (m, 1H), 1.98 - 1.94 (m, 3H). ¹³C NMR (201 MHz, CDCl₃) δ 157.60, 152.30, 133.85, 130.37, 128.85, 128.63, 127.46, 121.31, 120.54, 119.80, 119.73, 110.37, 63.98, 58.95, 56.50, 55.34, 49.10, 47.23, 40.93, 32.03, 30.61, 28.03, 25.72. HR-MS (ESI) *m*/*z* calculated for C₂₃H₂₉N₂OS⁺ [M+H]⁺: 381.1995, found: 381.1997. Specific rotation: [α]_{D}²⁵ = +142° (c = 0.1, CHCl₃).

### Example 26: Preparation of (8aS,12aR)-11-(3-(2-methoxyphenyl)propyl)-6,7,8a,9,10,11,12,12a-octahydro-5H-pyrido[4,3-b][1,4]thiazepino[2,3,4-hi]indole (compound I-26)

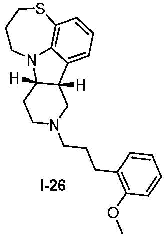

Following the method described in step 7 of Example 16, compound **WHF-21-p1** was subjected to Boc deprotection and then alkylated with 1-(3-bromopropyl)-2-methoxybenzene to obtain **I-26** (45 mg, yield of 50%) as an orange-yellow oil. ¹H NMR (800 MHz, CDCl₃) δ 7.18 - 7.16 (m, 1H), 7.12 (dd, *J =* 7.3, 1.7 Hz, 1H), 6.94 (dd, *J =* 7.8, 1.2 Hz, 1H), 6.90 - 6.81 (m, 3H), 6.64 - 6.59 (m, 1H), 3.85 - 3.79 (m, 4H), 3.56 - 3.52 (m, 1H), 3.28 - 3.26 (m, 1H), 3.21 - 3.17 (m, 1H), 3.07 - 3.04 (m, 1H), 2.95 - 2.92 (m, 1H), 2.83 - 2.77 (m, 1H), 2.71 - 2.68 (m, 1H), 2.61 (t, *J =* 7.7 Hz, 2H), 2.44 - 2.35 (m, 2H), 2.31 - 2.27 (d, *J =* 13.2 Hz, 1H), 2.13-2.09 (m, 1H), 2.05 - 1.98 (m, 1H), 1.97 - 1.88 (m, 3H), 1.84 - 1.80 (m, 2H). ¹³C NMR (201 MHz, CDCl₃) δ 157.51, 152.24, 133.72, 130.47, 129.92, 128.91, 127.18, 121.29, 120.45, 119.86, 119.77, 110.29, 63.95, 58.60, 56.42, 55.33, 49.11, 47.19, 40.79, 32.05, 30.61, 28.31, 26.92, 25.54. HR-MS (ESI) *m*/*z* calculated for C₂₄H₃₁N₂OS⁺ [M+H]⁺: 395.2152, found: 395.2152. Specific rotation: [α]_{D}²⁵ = -121° (c = 0.1, CHCl₃).

### Example 27: Preparation of (8aR,12aS)-11-(3-(2-methoxyphenyl)propyl)-6,7,8a,9,10,11,12,12a-octahydro-5H-pyrido[4,3-b][1,4]thiazepino[2,3,4-hi]indole (compound I-27)

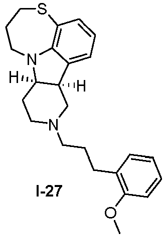

Following the method described in step 7 of Example 16, compound **WHF-21-p2** was subjected to Boc deprotection and then alkylated with 1-(3-bromopropyl)-2-methoxybenzene to obtain **I-27** (87 mg, yield of 94%) as an orange-yellow oil. ¹H NMR (800 MHz, CDCl₃) δ 7.17 (t, *J =* 7.8 Hz, 1H), 7.14 - 7.10 (m, 1H), 6.95 (d, *J =* 7.8 Hz, 1H), 6.90 - 6.82 (m, 3H), 6.62 (t, *J =* 7.5 Hz, 1H), 3.84 - 3.81 (m, 1H), 3.81 (s, 3H), 3.57 - 3.53 (m, 1H), 3.28 - 3.26 (m, 1H), 3.19 - 3.16 (m, 1H), 3.07 - 3.04 (m, 1H), 2.96 - 2.93 (m, 1H), 2.81 - 2.77 (m, 1H), 2.71 - 2.66 (m, 1H), 2.62 (t, *J =* 7.8 Hz, 2H), 2.42 - 2.34 (m, 2H), 2.28 - 2.24 (m, 1H), 2.13 - 2.09 (m, 1H), 2.04 - 1.99 (m, 1H), 1.95 - 1.87 (m, 3H), 1.83 - 1.79 (m, 2H). ¹³C NMR (201 MHz, CDCl₃) δ 157.51, 152.24, 133.83, 130.56, 129.90, 128.84, 127.13, 121.25, 120.42, 119.78, 119.70, 110.27, 64.01, 58.65, 56.54, 55.32, 49.17, 47.15, 40.90, 32.04, 30.60, 28.33, 27.05, 25.67. HR-MS (ESI) *m*/*z* calculated for C₂₄H₃₁N₂OS ⁺ [M+H]⁺: 395.2152, found: 395.2156. Specific rotation: [α]_{D}²⁵ = +130° (c = 0.1, CHCl₃).

### Example 28: Preparation of (6bS,10aR)-8-(2-methoxyphenyl)-3-methyl-2,3,6b,7,8,9,10,10a-octahydro-1H-pyrido[3',4':4,5]pyrrolo[1,2,3-de]quinoxaline (compound I-28)

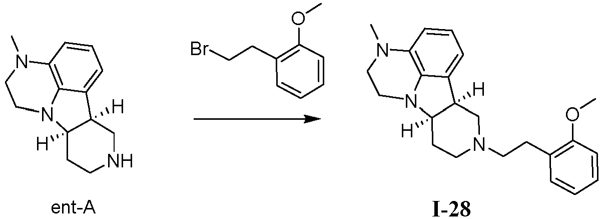

Following the method described in Example 3, compound **I-28** can be prepared using ent-A (prepared according to the methods described in Journal of Medicinal Chemistry, 2014, 57, 2670-2682 and Bioorganic & Medicinal Chemistry Letters, 13 (2003) 767-770) and 1-(2-bromoethyl)-2-methoxybenzene (CAS# 36449-75-9) as starting materials. ¹H NMR (800 MHz, CDCl₃) δ 7.18 (t, *J* = 7.4 Hz, 1H), 7.13 (d, *J* = 7.4 Hz, 1H), 6.86 (t, *J =* 7.4 Hz, 1H), 6.84 - 6.80 (m, 1H), 6.67 (t, *J =* 7.6 Hz, 1H), 6.54 (d, *J =* 7.3 Hz, 1H), 6.41 (d, *J =* 7.9 Hz, 1H), 3.78 (s, 3H), 3.62 - 3.58 (m, 1H), 3.36 - 3.32 (m, 1H), 3.31- 3.28 (m, 1H), 3.27 -3.24 (m, 2H), 3.10 (dd, *J =* 11.4, 6.4 Hz, 1H), 2.92 - 2.89 (m, 3H), 2.86 (s, 3H), 2.82 (t, *J =* 9.8 Hz, 1H), 2.72-2.63 (m, 2H), 2.53 - 2.48 (m, 1H), 2.20 - 2.10 (m, 2H), 2.04 - 1.97 (m, 1H). HR-MS (ESI) *m*/*z* calculated for C₂₃H₃₀N₃O⁺ [M+H]⁺: 364.2383; found: 364.2390. Specific rotation: [α]_{D}²⁵ = +53° (c = 0.1, CHCl₃).

### Example 29: Preparation of (6bS,10aR)-8-(3-(2-methoxy)propyl)-3-methyl-2,3,6b,7,8,9,10,10a-octahydro-1H-pyrido[3',4':4,5]pyrrolo[1,2,3-de]quinoxaline (compound I-29)

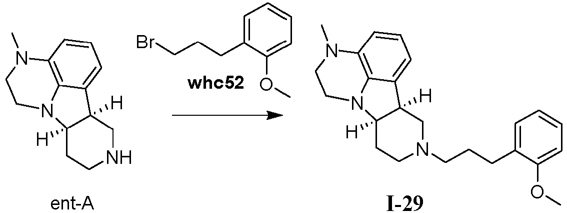

Following the method described in Example 10, compound **I-29** can be prepared as a yellow oil using ent-A (prepared according to the methods described in Journal of Medicinal Chemistry, 2014, 57, 2670-2682 and Bioorganic & Medicinal Chemistry Letters, 13 (2003) 767-770) and **whc52** as starting materials. ¹H NMR (800 MHz, CD₃OD) δ 7.23 - 7.18 (m, 1H), 7.14 (d, *J =* 7.4 Hz, 1H), 6.93 (d, *J =* 8.2 Hz, 1H), 6.87 (t, *J =* 7.4 Hz, 1H), 6.67 (d, *J =* 9.4 Hz, 1H), 6.58 (d, *J =* 7.2 Hz, 1H), 6.53 (d, *J =* 8.0 Hz, 1H), 3.82 (s, 3H), 3.57 - 3.52 (m, 2H), 3.47 - 3.43 (m, 1H), 3.43 - 3.40 (m, 1H), 3.39 - 3.32 (m, 2H), 3.29 - 3.25 (m, 1H), 3.19 - 3.15 (m, 1H), 3.12 - 3.04 (m, 2H), 2.88 (s, 3H), 2.84 - 2.80 (m, 1H), 2.69 (t, *J =* 7.4 Hz, 2H), 2.65 (t, *J =* 12.4 Hz, 1H), 2.36 - 2.31 (m, 1H), 2.16 - 2.09 (m, 1H), 2.07 - 2.00 (m, 2H). HR-MS (ESI) *m*/*z* calculated for C₂₄H₃₂N₃O⁺ [M+H]⁺: 378.2540; found: 378.2545. Specific rotation: [α]_{D}²⁵ = +40° (c = 0.1, CHCl₃).

### Biological Test Example 1: Assessing the affinity of the compounds of the present disclosure for the 5-HT_{2A} receptor

Method: The affinity of the compounds of the present disclosure for the 5-HT_{2A} receptor was determined using a radioligand competitive binding assay.

In the first step, a cell membrane component containing 5-HT_{2A} receptor was prepared as follows. HEK-293T cells (ATCC, CRL-11268) in a 10-cm dish were transfected with 10 ng of 5-HT_{2A} receptor plasmid and 40 µL of PEI. The 10-cm dish was removed from the incubator 48 hours post-transfection, a time point by which the cultured cells had expressed 5-HT_{2A} receptor protein.. The culture medium was aspirated using a vacuum pump, and 3 mL of lysis buffer was added to each dish. The cells were then placed in a cold room at 4°C for 10 minutes. After the cells were detached, they were transferred to a 15 mL centrifuge tube and centrifuged at 1500 rpm for 5 minutes at 4°C, and the supernatant was discarded. The cell pellet was transferred to a tissue homogenizer, and 3 mL of lysis buffer was added. Sufficient grinding was applied to break the cells. The cell suspension was then equally aliquoted into several Eppendorf tubes and centrifuged at 12,000 rpm for 5 minutes at 4°C, and the supernatant was discarded. The resulting precipitate was the cell membrane component containing the 5-HT_{2A} receptor.

In the second step, a ligand-receptor binding assay was performed on the 293T membrane component transiently expressing the 5-HT_{2A} receptor. First, standard binding buffer was added to the cell membrane component containing the 5-HT_{2A} receptor, and the cell membrane was disrupted and resuspended with an electric tissue homogenizer. 30 µL of membrane protein suspension was added to each well of a 96-well plate. Then, 30 µL of different drugs were added to the 96-well plate sequentially from left to right, ensuring that the final drug concentrations were 10⁻⁵ M, 10⁻⁶ M, 10⁻⁷ M, 10⁻⁸ M, 10⁻⁹ M, and 0 M from bottom to top, with two replicates per treatment. Next, 30 µL of [³H]-LSD was added to each well of the 96-well plate. The plate was then incubated at room temperature in the dark for 2 hours. Detection was carried out. The machine readout value reflected the amount of [³H]-LSD bound on the membrane, and after further data processing, the affinity Kᵢ values of different compounds for the 5-HT_{2A} receptor were obtained.

Results: The affinity Kᵢ values of the compounds of the present disclosure for the 5-HT_{2A} receptor are shown in Table 1.

**Table 1**

| Example compounds | Kᵢ, µM |
|---|---|
| **I-1** | 0.246 |
| **I-2** | 0.525 |
| **I-3** | 0.017 |
| **I-4** | 0.088 |
| **I-5** | 0.126 |
| **I-6** | 5.245 |
| **I-7** | 1.581 |
| **I-8** | 3.059 |
| **I-9** | 0.031 |
| **I-10** | 0.013 |
| **I-11** | 0.089 |
| **I-12** | 0.190 |
| **I-13** | 0.034 |
| **I-14** | 0.028 |
| **I-15** | 0.007 |
| **I-16** | 0.065 |
| **I-17** | 0.024 |
| **I-18** | 0.057 |
| **I-19** | 0.018 |
| **I-20** | 0.107 |
| **I-21** | 0.043 |
| **I-22** | 0.099 |
| **I-23** | 0.011 |
| **I-24** | 0.045 |
| **I-25** | 0.032 |
| **I-26** | 0.093 |
| **I-27** | 0.008 |
| **I-28** | \ |
| **I-29** | \ |

As can be seen from Table 1, the compounds of the present disclosure exhibit moderate to high affinity for the 5-HT_{2A} receptor, with compounds I-3, I-4, I-9, I-10, I-11, I-12, I-13, I-14, 1-15, 1-16, 1-17, 1-18, 1-19, 1-20, 1-21, 1-22, 1-23, 1-24, 1-25, 1-26, and 1-27 having Kᵢ values less than 0.1 µM for the 5-HT_{2A} receptor.

### Biological Test Example 2: Functional activity testing of the compounds of the present disclosure on the 5-HT_{2A} receptor

Method: To detect the downstream G protein signaling pathway mediated by the 5-HT_{2A} receptor, on the first day, HEK-293T cells (ATCC, CRL-11268) spread in a 6 cm culture dish were transfected with 1 µg of 5-HT_{2A} receptor plasmid, 1 µg of Gαq containing C-terminal Renilla luciferase (Gαq-Rluc), 1 µg of Gβ3, 1 µg of Gy9 containing C-terminal green fluorescent protein (Gγ9-GFP), and 16 µL of PEI. In parallel, to detect the downstream β-arrestin2 signaling pathway mediated by the 5-HT_{2A} receptor, on the first day, HEK-293T cells (ATCC, CRL-11268) spread in a 6 cm culture dish were transfected with 500 µg of 5-HT_{2A} receptor plasmid containing C-terminal Renilla luciferase (5-HT_{2A}-Rluc), 500 µg of G protein-coupled receptor kinase 2 (GRK2), 2500 µg of β-arrestin2 containing N-terminal green fluorescent protein (GFP2-ARRB2), and 14 µL of PEI. On the second day, the confluent cells from a 6 cm culture dish were digested and reseeded into a 96-well plate at a volume of 100 µL of culture medium per well. On the third day, the drugs were added for testing. The 96-well plate was taken out from the cell incubator to decant the culture medium. 40 µL of the substrate coelenterazine 400a (final concentration 5 µM) was added to each well, and then 20 µL of different drugs were added sequentially from left to right, ensuring that the final concentration of the drug decreased gradually from bottom to top, with two replicates per treatment. Finally, the prepared samples were loaded on a plate reader for signal quantification. The readout values reflected the recruitment of β-arrestin2 to the membrane or the dissociation of G protein trimer. The former indicated the degree of activation of the downstream β-arrestin2 signaling pathway mediated by the 5-HT_{2A} receptor, and the latter indicated the degree of activation of the downstream G protein signaling pathway mediated by the 5-HT_{2A} receptor. Thus, the agonistic effects of different compounds on the 5-HT_{2A} receptor can be revealed.

Results: The agonistic activity of the compounds of the present disclosure on the 5-HT_{2A} receptor is shown in Table 2 ("NA" indicates no agonistic activity; "\" indicates no testing conducted).

**Table 2**

| Example compounds | Gq signaling agonistic activity | β-arrestin2 signaling agonistic activity |
|---|---|---|
| I-1 | 120.2 nM (67.0%) | 173.8 nM (60.2%) |
| I-2 | 309.0 nM (65.3%) | 229.1 nM (68.4%) |
| I-3 | 707.9 nM (20.2%) | 173.8 nM (39.0%) |
| I-4 | 185.2 nM (13.7%) | 213.7 nM (44.2%) |
| I-5 | NA | 683.7 nM (3.2%) |
| I-6 | \ | \ |
| I-7 | \ | \ |
| I-8 | \ | \ |
| I-9 | NA | 75.86 nM (14.4%) |
| I-10 | NA | 204.2 nM (12.7%) |
| I-11 | 371.5 nM (9.7%) | 229.1 nM (36.8%) |
| **I-12** | NA | NA |
| **I-13** | NA | 181.1 nM (51.4%) |
| **I-14** | NA | NA |
| **I-15** | NA | 97.24 nM (24.3%) |
| **I-16** | NA | NA |
| **I-17** | NA | 718.9 nM (64.6%) |
| **I-18** | NA | NA |
| **I-19** | NA | 561.7 nM (59.4%) |
| **I-20** | NA | NA |
| **I-21** | NA | 582.5 nM (40.6%) |
| **I-22** | NA | NA |
| **I-23** | NA | 226.6 nM (51.6%) |
| **I-24** | NA | NA |
| **I-25** | NA | 988.0 nM (43.6%) |
| **I-26** | NA | NA |
| **I-27** | NA | 878.4 nM (35.7%) |
| **I-28** | \ | \ |
| **I-29** | \ | \ |

As can be seen from Table 2, the compounds of the present disclosure exhibit moderate to strong agonistic activity on the 5-HT_{2A} receptor. Furthermore, compounds 1-9,1-10,1-13, 1-15, 1-17, 1-19, 1-21, 1-23, 1-25, and 1-27 selectively activate the recruitment of β-arrestin2 mediated by the 5-HT_{2A} receptor, without activating the G protein signaling pathway.

### Biological Test Example 3: Selectivity testing of compounds I-3 and I-10 for the dopamine D₂ receptor

Method: The affinity of the compounds of the present disclosure for the dopamine D₂ receptor was determined using a radioligand competitive binding assay as follows. In the first step, a cell membrane component containing dopamine D₂ receptor was prepared. HEK-293T cells (ATCC, CRL-11268) spread in a 10 cm culture dish were transfected with 10 ng of dopamine D₂ receptor plasmid and 40 µL of PEI. 48 hours post-transfection, the 10 cm culture dish was taken out from the cell incubator and the cultured cells had expressed the dopamine D₂ receptor. The culture medium was aspirated using a vacuum pump, and 3 mL of lysis buffer was added to each culture dish. The cells were then placed in a cold room at 4°C for 10 minutes. After the cells were detached, they were transferred to a 15 mL centrifuge tube and centrifuged at 1500 rpm for 5 minutes at 4°C, and the supernatant was discarded. The cell pellet was transferred to a tissue homogenizer, and 3 mL of lysis buffer was added. Sufficient grinding was applied to break the cells. The cell suspension was then equally aliquoted into several Eppendorf tubes and centrifuged at 12000 rpm for 5 minutes at 4°C, and the supernatant was discarded. The resulting precipitate was the cell membrane component containing the dopamine D₂ receptor. In the second step, a ligand-receptor binding assay was performed on the 293T membrane component transiently expressing the dopamine D₂ receptor. First, standard binding buffer was added to the cell membrane component containing the dopamine D₂ receptor, and the cell membrane was disrupted and resuspended with an electric tissue homogenizer. 30 µL of membrane protein suspension was added to each well of a 96-well plate. Then, 30 µL of different drugs were added to the 96-well plate sequentially from left to right, ensuring that the final drug concentrations were 10⁻⁵ M, 10⁻⁶ M, 10⁻⁷ M, 10⁻⁸ M, 10⁻⁹ M, and 0 M from bottom to top, with two replicates per treatment. Next, 30 µL of [³H]-Methylspiperone was added to each well of the 96-well plate. The plate was then incubated at room temperature in the dark for 2 hours. Detection was carried out. The machine readout value reflected the amount of [³H]-Methylspiperone bound on the membrane, and after further data processing, the affinity Kᵢ values of different compounds for the dopamine D₂ receptor were obtained.

Results: The affinity of compound 1-3 for the dopamine D₂ receptor was characterized by Kᵢ of 1.55 µM, and the affinity of compound 1-10 for the dopamine D₂ receptor was characterized by Kᵢ of 1.86 µM. Therefore, compounds 1-3 and 1-10 exhibit weak affinity for the dopamine receptor. Compared to lumateperone, compounds 1-3 and 1-10 show better selectivity for the 5-HT_{2A} receptor.

### Biological Test Example 4: Testing of metabolic properties of compounds I-10 and I-23 in mice

Compound **I-10** was administered intraperitoneally in a single dose to male mice, and blood samples were collected at different time points. The concentration of the compound in mouse plasma was determined using LC-MS/MS, and the relevant pharmacokinetic parameters were calculated to investigate the pharmacokinetic characteristics of the compound in mice.

Experimental design: 60 male C57 mice (purchased from Suzhou JOINN Laboratory Animal Co., Ltd.) were randomly divided into groups based on weight, with 3 mice per group. The mice were fasted with water *ad libitum* for 12 to 14 hours one day before test-article administration and fed 4 hours after the administration. The route of administration was intraperitoneal (IP), with a dosing concentration of 1 mg/mL, and a solvent formulation of 10% DMSO + 90% saline.

Sample collection: 0.1 mL of blood was collected from the orbit under isoflurane anesthesia before and after test-article administration, transferred to EDTA K2 tubes, and placed on an ice bath. The samples were centrifuged at 5000 rpm for 10 minutes at 4°C, and the plasma was collected. The blood and brain tissue collection time points were 0 min, 5 min, 15 min, 30 min, 1 hour, 2 hours, 4 hours, 6 hours, 8 hours, and 24 hours, with three mice per time point. Prior to analysis, all plasma samples were stored at -80°C.

Data processing: Drug concentrations in plasma and brain tissues were measured using LC-MS (AP14000QTRAP triple quadrupole tandem mass spectrometer, equipped with an electrospray ionization source (ESI)). The data collection and control system software utilized was Analyst 1.5.1 (Applied Biosystems). The sample peak integration in the spectrum was conducted automatically; the ratio of the sample peak area to the internal standard peak area was used as an indicator to perform regression with the concentration of the sample. Regression method: Linear regression with a weighting factor of 1/X². Pharmacokinetic parameters were analyzed using a non-compartmental model in WinNonlin Professional v6.3 (Pharsight, USA). C*ₘₐₓ* is the measured maximum plasma concentration, the area under the plasma concentration-time curve (AUC*_{(0→t)}*) was calculated by the trapezoidal method, and T*ₘₐₓ* is the time to reach peak plasma concentration after administration. Experimental data are presented as "Mean ± Standard Deviation" (Mean ± SD, n ≥ 3) or "Mean" (n = 2).

Experimental results: The pharmacokinetic parameters for compounds **I-10** and **I-23** (via intraperitoneal administration) are shown in Table 3 below:

**Table 3**

| **Pharmacokinetic parameters** | | **I-10** (10 mg/kg) | | | **I-23** (5 mg/kg) | | |
|---|---|---|---|---|---|---|---|
| | | **Plasma** | **Brain** | **Brain/ plasma ratio** | **Plasma** | **Brain** | **Brain/ plasma ratio** |
| Kₑₗ | h⁻¹ | 0.654 | 0.865 | 2.32 | 0.678 | 0.630 | 3.38 |
| T_{1/2} | h | 1.06 | 0.801 | | 1.02 | 1.10 | |
| tₘₐₓ | h | 0.083 | 0.500 | | 0.50 | 0.50 | |
| Cₘₐₓ | ng·mL⁻¹ or ng·g⁻¹ | 225 | 566 | | 272 | 1417 | |
| AUC₀₋ₜ | h·ng·mL⁻¹ or ng·g⁻¹ | 576 | 1335 | | 537 | 1816 | |
| AUC_{0-inf} | h·ng·mL⁻¹ or ng·g⁻¹ | 582 | 1346 | | 540 | 1822 | |
| AUMC ₀₋ₜ | h·h·ng·mL⁻¹ or ng·g⁻¹ | 1169 | 1979 | | 769 | 1645 | |
| AUMC _{0-inf} | h·h·ng·mL⁻¹ or ng·g⁻¹ | 1230 | 2054 | | 794 | 1698 | |
| MRT | h | 2.11 | 1.53 | | 1.47 | 0.932 | |

### Biological Test Example 5: Antidepressant activity test of compounds I-3 and I-10

Method: Acute restraint stress (ARS) is frequently used to induce a depressive state in mice. The forced swim test (FST) and tail suspension test (TST) are currently the most commonly used models for inferring "depression-like" behaviors. C57BL/6J wild-type mice (over 8 weeks old, purchased from Shanghai Lingchang Biotech Co., Ltd.) were restrained individually in a rodent restraint device for 5 hours. The mice were restricted from all body movements, but only minimal pain was introduced. During the ARS experiment, the mice were deprived of both food and water. After 5 hours of restraint, a separate group of mice was released and immediately injected intraperitoneally with the test compound. After 30 minutes of recovery, the behavioral changes of the mice were assessed through the Forced Swim Test (FST) or Tail Suspension Test (TST). The control group received saline injections but had not undergone the ARS procedure. In the FST, mice in the control group and those subjected to ARS were placed separately in a glass beaker containing 15 cm deep water at a temperature of 25 ± 1°C. All mice were forced to swim for 6 minutes, and their behavior was recorded using a video camera. The immobility time during the last 4 minutes of the test was recorded. Immobility time was defined as the time during which the animal floats in the water without struggling, making only the movements necessary to keep its head above water. In the TST, mice in the control group and those subjected to restraint stress were suspended by their tails with tape for 6 minutes, and their behavior was recorded using a video camera. The immobility time during the last 4 minutes was manually timed and assessed by an observer who was unaware of the treatment conditions.

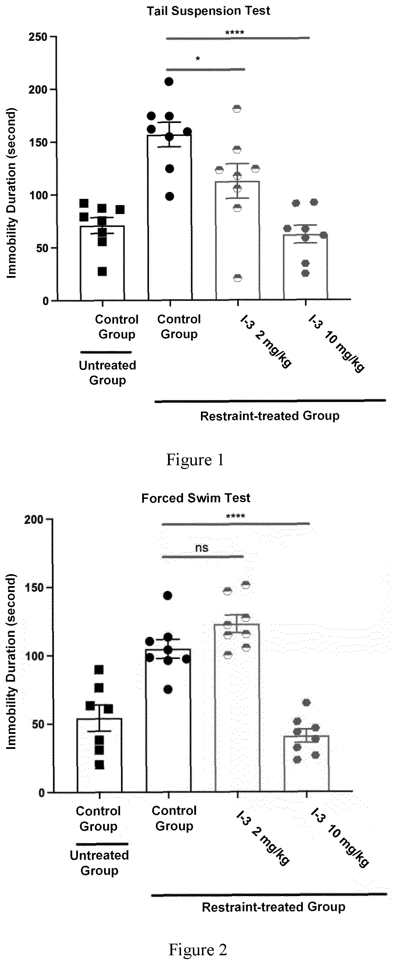

Results: The antidepressant effect of compound 1-3 in the TST is shown in Figure 1. As can be seen from Figure 1, compound 1-3 has a significant antidepressant effect. The antidepressant effect of compound 1-3 in the FST is shown in Figure 2. As can be seen from Figure 2, compound 1-3 has a significant antidepressant effect.

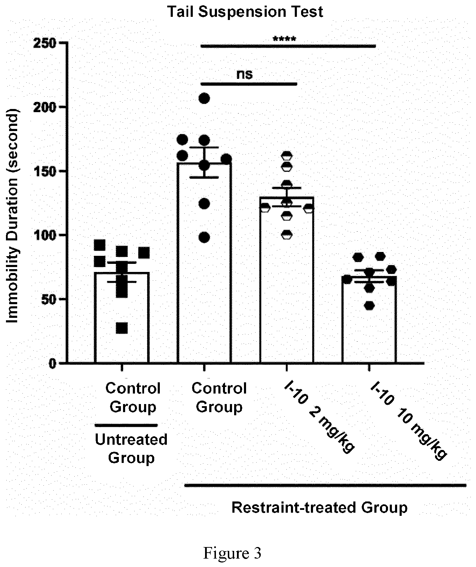

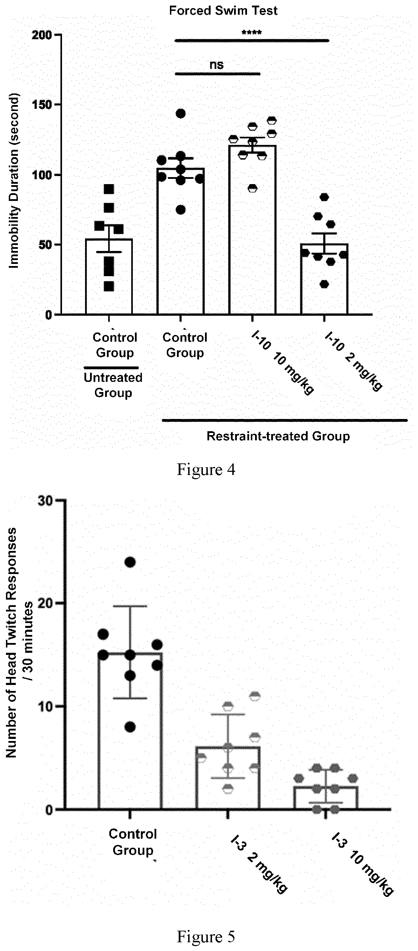

The antidepressant effect of compound 1-10 in the TST is shown in Figure 3. As can be seen from Figure 3, compound 1-10 has a significant antidepressant effect. The antidepressant effect of compound 1-10 in the FST is shown in Figure 4. As can be seen from Figure 4, compound 1-10 has a significant antidepressant effect.

### Biological Test Example 6: Testing of hallucinogenic effects of compounds I-3 and I-10

Method: The head-twitch response in mice was detected via oscillatory signals to characterize hallucinogenic effects. Mice (strain C57BL/6J, purchased from Shanghai Lingchang Biotech Co., Ltd.) were anesthetized with 2% isoflurane. A small portion of the scalp was removed from the center of the dorsal cranium. A custom-made small neodymium magnet (4 mm × 4 mm × 2 mm) was attached to the center of the dorsal cranium using dental resin. The magnet N-S poles were kept parallel to the dorsoventral aspect of the implant. The mice were allowed to recover for 1 week before testing. To record precise head movements, the mice were placed in a glass beaker with a diameter of 12 cm, surrounded by 150 turns of #30 enameled copper wire. The output signal from the coil was recorded using a PowerLab/4SP instrument installed with LabChart V8.1.16 software. Coil voltage was amplified, low-pass filtered at 10 kHz to remove radio frequency interference, and sampled at 40 kHz. For the detection of head-twitch response in mice, LabChart data were processed with a digital band-pass filter at 40 to 200 Hz. The head-twitch response in mice was identified by searching for sinusoidal wavelets that met the following criteria: 1) containing more than two bipolar peaks; 2) amplitude exceeding background noise level; 3) duration less than 120 milliseconds. 1 hour prior to the test, the mice were transferred to the test room for acclimatization. Subsequently, the mice were placed in the beaker for 30 minutes, and baseline head-twitch response values were recorded. Immediately after baseline recording, the mice were injected intraperitoneally with the test compound, and head-twitch response values were recorded for 1 to 2 hours post-dose, depending on the compound used. Statistical values were taken from the first 30 minutes.

Results: The head-twitch response in mice treated with compound 1-3 is shown in Figure 5. As can be seen from Figure 5, compound 1-3 did not induce hallucinogenic effects in mice at both doses tested.

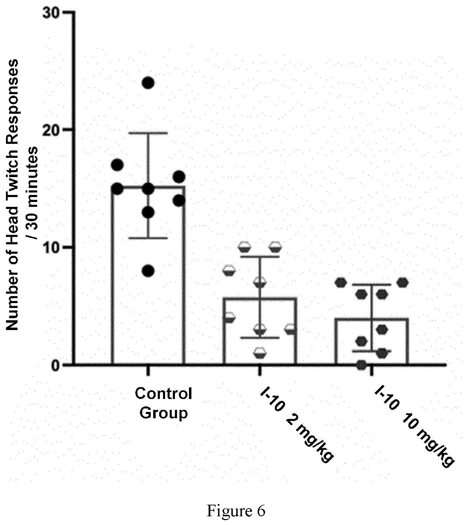

The head-twitch response in mice treated with compound 1-10 is shown in Figure 6. As can be seen from Figure 6, compound 1-10 also did not induce hallucinogenic effects in mice at both doses tested.

### Biological Test Example 7: Testing of antidepressant and hallucinogenic effects of compound I-23

Antidepressant efficacy test method: Male C57BL/6 mice (8 weeks old, purchased from Shanghai Lingchang Biotech Co., Ltd.) were administered a solution of corticosterone in their drinking water at a concentration of 25 µg/mL using drinking water bottles for 21 days. Mice were maintained on a 12-hour light cycle. Corticosterone-containing drinking water was refreshed every other day for the first two weeks, and gradually replaced with corticosterone-free drinking water every two days for the last week. Compound **I-23** was administered to the mice via a single injection after 21 days of corticosterone exposure, and the antidepressant activity of compound **I-23** was assessed through the TST at 1 day, 7 days, and 14 days after injection. TST procedure: mice in the control group and the administration group were suspended by their tails with tape for 6 minutes, and their behavior was recorded by a video camera. The immobility time during the last 4 minutes was manually timed and assessed by an observer who was unaware of the treatment conditions.

Hallucinogenic effect of the test compound I-23 was assessed via the head-twitch response, using the same method as described in Biological Test Example 6.

### Results:

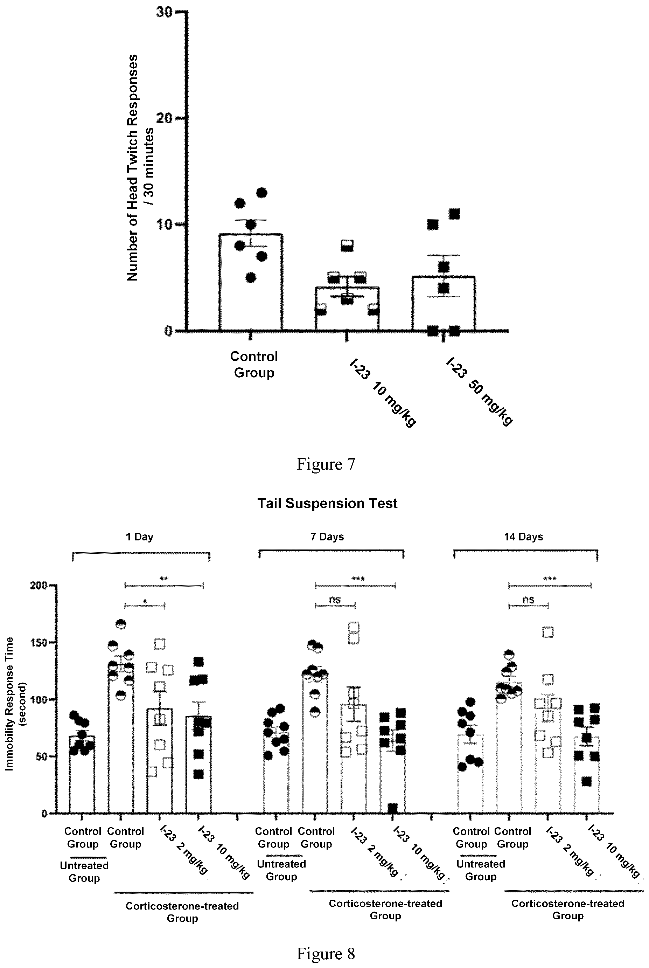

The head-twitch response in mice treated with compound **I-23** is shown in Figure 7. As can be seen from Figure 7, compound **I-23** did not induce hallucinogenic effects in mice at any of the doses tested.

The antidepressant effect of compound **I-23** in the mouse tail suspension test is shown in Figure 8. As can be seen from Figure 8, compound **I-23** had a significant antidepressant effect one day after a single administration, and the antidepressant effect remained significant in the mice of the 10 mg/kg single-injection group after 7 and 14 days.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A fused-piperidine compound of formula **I'** or a pharmaceutically acceptable salt thereof: 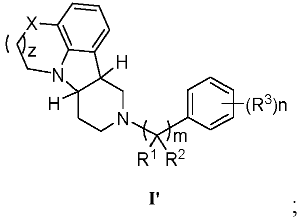
wherein X is 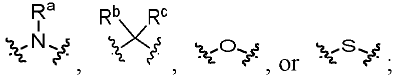 R^{a}, R^{b}, and R^{c} are independently hydrogen or C₁-C₄ alkyl;
R¹ and R² are independently hydrogen, halogen, or C₁-C₄ alkyl;
R³ is independently hydrogen, halogen, hydroxyl, or C₁-C₄ alkoxy;
m is 1, 2, or 3;
n is 1, 2, or 3;
z is 1 or 2.

2. The fused-piperidine compound of formula **I'** according to claim 1 or a pharmaceutically acceptable salt thereof, wherein the fused-piperidine compound of formula **I'** is a fused-piperidine compound of formula 1-a, 1-b, I, or I-c: 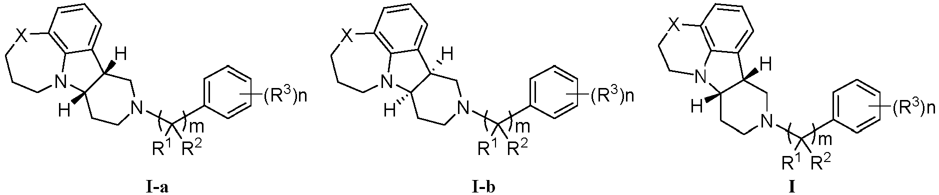 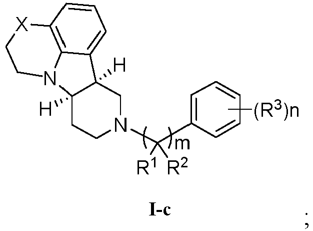
wherein X, R¹, R², R³, m, and n are as defined in claim 1;
for example, the fused-piperidine compound of formula **I'** is a fused-piperidine compound of formula I: 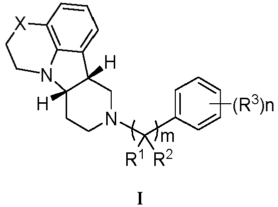
wherein X is 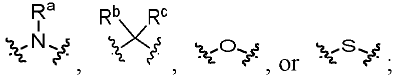 R^{a}, R^{b}, and R^{c} are independently hydrogen or C₁-C₄ alkyl;
R¹ and R² are independently hydrogen, halogen, or C₁-C₄ alkyl;
R³ is independently hydrogen, halogen, hydroxyl, or C₁-C₄ alkoxy;
m is 1, 2, or 3;
n is 1, 2, or 3.

3. The fused-piperidine compound of formula **I'** according to claim 1 or a pharmaceutically acceptable salt thereof, wherein in R^{a}, R^{b}, and R^{c}, the C₁-C₄ alkyl is independently methyl, ethyl, *n*-propyl, isopropyl, *n*-butyl, isobutyl, *sec*-butyl, or *tert*-butyl, for example, methyl;
and/or, in R¹ and R², the halogen is independently F, Cl, Br, or I;
and/or, in R¹ and R², the C₁-C₄ alkyl is independently methyl, ethyl, *n*-propyl, isopropyl, *n*-butyl, isobutyl, *sec*-butyl, or *tert*-butyl;
and/or, in R³, the halogen is independently F, Cl, Br, or I;
and/or, in R³, the C₁-C₄ alkoxy is methoxy, ethoxy, *n*-propoxy, isopropoxy, *n*-butoxy, isobutoxy, *sec*-butoxy, or *tert*-butoxy, for example, methoxy;
and/or, the substitution position of R³ on the benzene ring is *ortho*-position, *"ortho-* and *para*-position", or *"ortho-* and *meta*-position".

4. The fused-piperidine compound of formula **I'** according to claim 1 or a pharmaceutically acceptable salt thereof, wherein X is 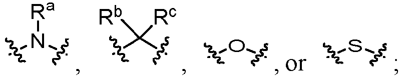 for example, X is 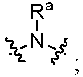
and/or, R^{a} is C₁-C₄ alkyl;
and/or, R^{b} and R^{c} are independently hydrogen;
and/or, R¹ and R² are hydrogen;
and/or, R³ is independently hydrogen, hydroxyl, or C₁-C₄ alkoxy;
and/or, n is 1 or 2.

5. The fused-piperidine compound of formula **I'** according to claim 1 or a pharmaceutically acceptable salt thereof, wherein X is 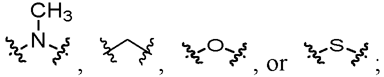 for example, X is 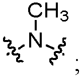 and/or, 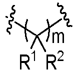 is 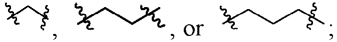 and/or, 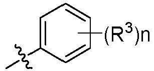 is

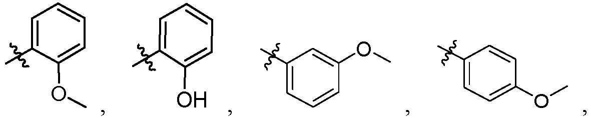

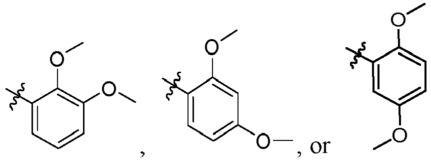

6. The fused-piperidine compound of formula **I'** according to claim 5 or a pharmaceutically acceptable salt thereof, wherein 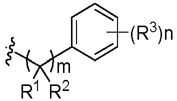 is

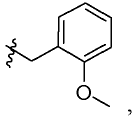

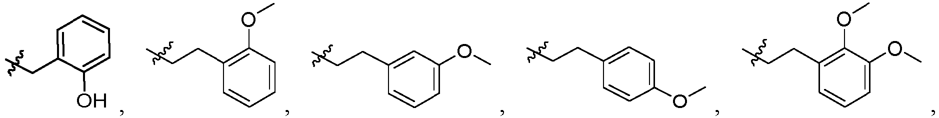

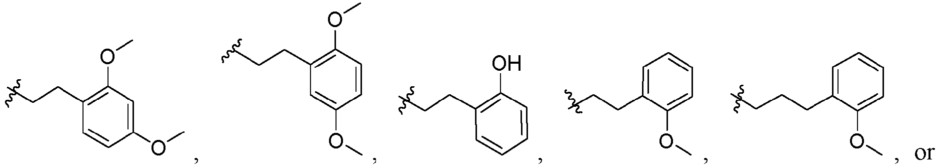

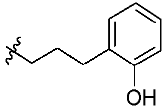

7. The fused-piperidine compound of formula **I'** according to claim 1 or a pharmaceutically acceptable salt thereof, wherein the fused-piperidine compound of formula **I'** conforms to any one of the following schemes:
scheme 1:
Xis 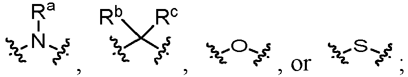 R^{a} is C₁-C₄ alkyl;
R^{b} and R^{c} are independently hydrogen;
R¹ and R² are hydrogen;
R³ is independently hydrogen, hydroxyl, or C₁-C₄ alkoxy;
n is 1 or 2;
z is 1 or 2;
the substitution position of R³ on the benzene ring is *ortho*-position, *"ortho-* and *para-*position", or *"ortho-* and *meta*-position";
scheme 2:
Xis 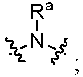
R^{a} is C₁-C₄ alkyl;
R¹ and R² are hydrogen;
R³ is independently hydrogen, hydroxyl, or C₁-C₄ alkoxy;
n is 1 or 2;
the substitution position of R³ on the benzene ring is *ortho*-position, *"ortho-* and *para-*position", or *"ortho-* and *meta*-position".

8. The fused-piperidine compound of formula **I'** according to claim 1 or a pharmaceutically acceptable salt thereof, wherein the fused-piperidine compound of formula **I'** is any one of the following compounds:

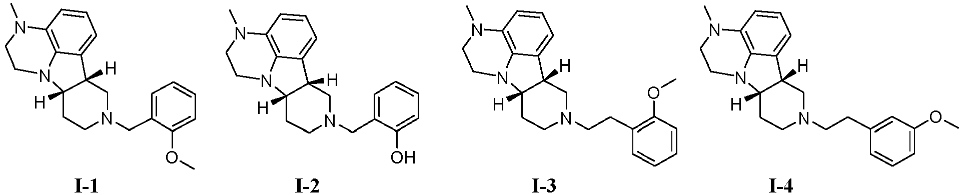

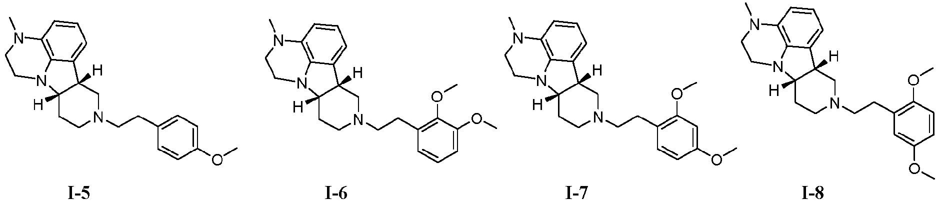

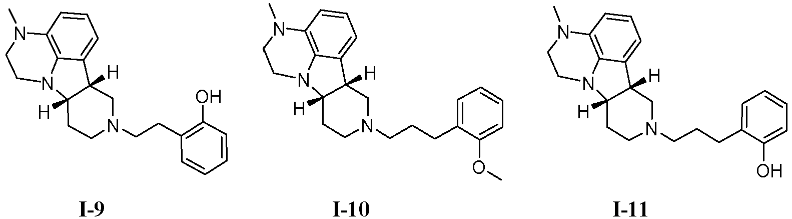

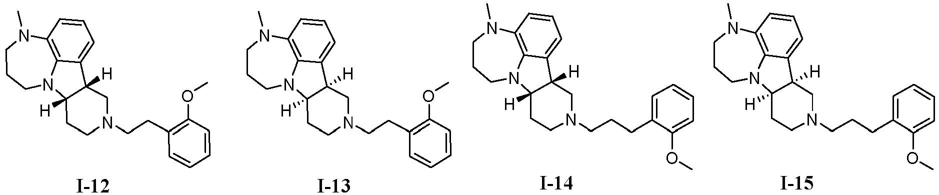

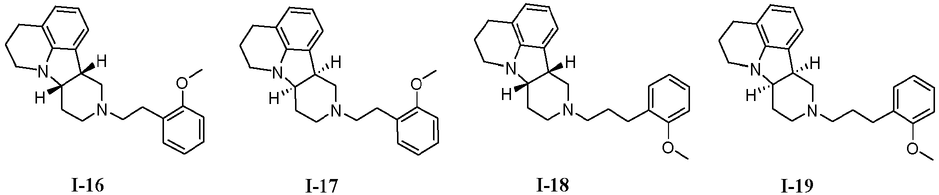

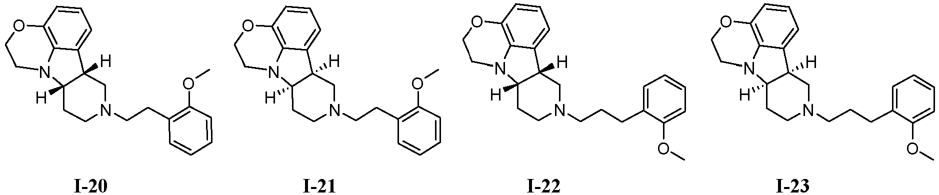

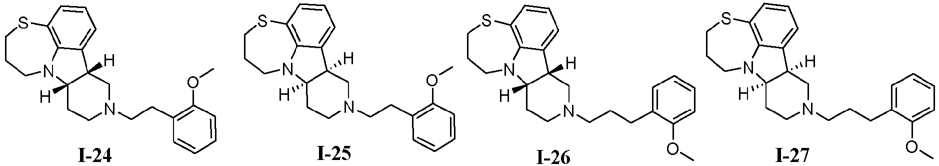

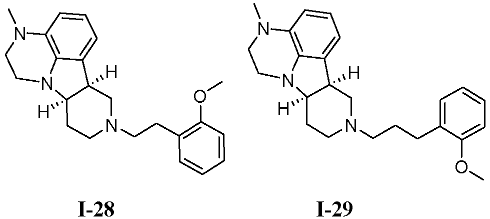

9. A preparation method for the fused-piperidine compound of formula **I'** according to any one of claims 1 to 8 or a pharmaceutically acceptable salt thereof, comprising Method **1,** Method **2,** or Method **3:**
when R³ is independently hydrogen, halogen, or C₁-C₄ alkoxy, the preparation method for the fused-piperidine compound of formula **I'** or the salt thereof is Method **1;**
Method **1** comprises the following steps: in the presence of a base, conducting a substitution reaction in a solvent between a compound of formula **II'** and a compound of formula **III** to obtain the fused-piperidine compound of formula **I';** 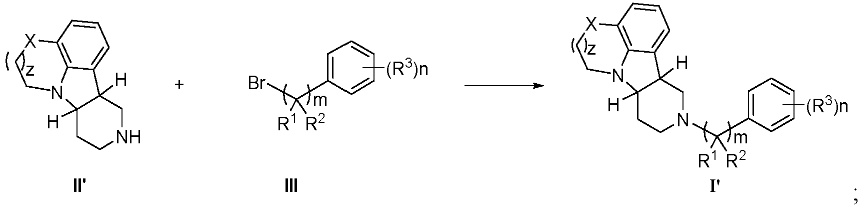
Method 2 comprises the following steps: in the presence of a reducing agent, conducting a reductive amination reaction in a solvent between a compound of formula **II'** and a compound of formula **IV** to obtain the fused-piperidine compound of formula **I';** 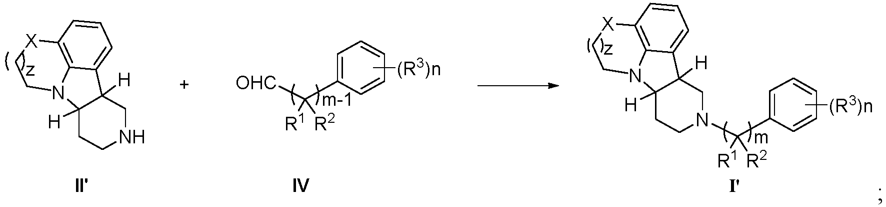
when R³ is independently hydroxyl, the preparation method for the fused-piperidine compound of formula **I'** or the salt thereof is Method **3;**
Method 3 comprises the following steps: in the presence of BBrs, reacting the fused-piperidine compound of formula **I'** as described in method **1** or **2** in a solvent to obtain the fused-piperidine compound of formula **I'** (*i.e.,* R³ is hydroxyl);
in Methods **1, 2,** and **3,** X, R^{a}, R^{b}, R^{c}, R¹, R², m, and n are as defined in any one of claims 1 to 8;
preferably, the preparation method satisfies the following conditions:
in Method **1,** the base is preferably an organic base, for example, diisopropylethylamine;
and/or, in Method **1,** the molar ratio of the base to the compound of formula **II'** is preferably (1 to 3): 1, for example, 1:1;
and/or, in Method **1,** the molar ratio of the compound of formula **III** to the compound of formula **II'** is preferably (1 to 3): 1, for example, 1.5:1;
and/or, in Method **1,** the solvent is preferably a polar aprotic organic solvent, for example, dimethyl sulfoxide;
and/or, in Method **1,** the temperature for the substitution reaction is preferably room temperature to 80°C, more preferably 50°C to 80°C, for example, 60°C;
and/or, in Method **2,** the solvent is preferably an alcoholic solvent, for example, methanol;
and/or, in Method **2,** the reducing agent is preferably a borane reducing agent, for example, sodium cyanoborohydride;
and/or, in Method **2,** the temperature for the reaction is preferably 0 to 60°C, for example, room temperature;
and/or, in Method **3,** the solvent is preferably a halogenated hydrocarbon organic solvent, for example, dichloromethane;
and/or, in Method **3,** the temperature for the reaction is preferably -40°C to 40°C, more preferably 10°C to 40°C, for example, room temperature.

10. A pharmaceutical composition comprising the fused-piperidine compound of formula **I'** according to any one of claims 1 to 8 or a pharmaceutically acceptable salt thereof, and a pharmaceutically acceptable excipient.

11. A use of a substance **A** in the preparation of a 5-HT_{2A} receptor agonist or a medicament;
the substance **A** is the fused-piperidine compound of formula **I'** according to any one of claims 1 to 8 or a pharmaceutically acceptable salt thereof, or the pharmaceutical composition according to claim 10;
the medicament is a medicament for treating or preventing a disease associated with the 5-HT_{2A} receptor.

12. The use according to claim 11, wherein the 5-HT_{2A} receptor agonist is a 5-HT_{2A} receptor agonist of the downstream β-arrestin2 recruitment signaling pathway and/or a 5-HT_{2A} receptor agonist of the downstream Gq protein activation signaling pathway, preferably a 5-HT_{2A} receptor agonist of the downstream β-arrestin2 recruitment signaling pathway;
and/or, the disease associated with the 5-HT_{2A} receptor is a disease associated with the downstream β-arrestin2 recruitment signaling pathway of the 5-HT_{2A} receptor and/or the downstream Gq protein activation signaling pathway of the 5-HT_{2A} receptor, preferably a disease associated with the downstream β-arrestin2 recruitment signaling pathway of the 5-HT_{2A} receptor, for example, depression.

13. A use of a substance **A** in the preparation of a medicament for treating or preventing depression, wherein the substance **A** is the fused-piperidine compound of formula **I'** according to any one of claims 1 to 8 or a pharmaceutically acceptable salt thereof, or the pharmaceutical composition according to claim 10.
